# EUROPEAN PATENT SPECIFICATION

(11) **EP 2 691 544 B1**
(45) Date of publication and mention of the grant of the patent: **13.09.2017**
(21) Application number: 12764565.3
(22) Date of filing: 30.03.2012
(51) Int. Cl.: C12Q 1/68

(54) **METHOD FOR VERIFYING BIOASSAY SAMPLES**
VERFAHREN ZUR PRÜFUNG VON BIOASSAYPROBEN
PROCÉDÉ DE VÉRIFICATION D'ÉCHANTILLONS DE BIOANALYSE

(30) Priority: 30.03.2011 US 201161469236 P
(43) Date of publication of application: 05.02.2014
(73) Proprietor: Verinata Health, Inc., Redwood City, CA 94063 (US)
(72) Inventor: COMSTOCK, David, A., San Francisco, CA 94107 (US); SRINIVASAN, Anupama, Redwood City, CA 94061 (US)
(74) Representative: Chapman, Desmond Mark
(86) International application number: PCT/US2012/031625
(87) International publication number: WO 2012/135730

(56) References cited:
- WO-A1-96/19586
- WO-A1-98/14275
- WO-A2-2008/093098
- WO-A2-2010/075188
- US-A1- 2009 029 377
- US-A1- 2010 035 246
- US-A1- 2010 136 529
- US-A1- 2011 039 724
- US-B1- 6 673 621
- R. W. K. Chiu ET AL: "Non-invasive prenatal assessment of trisomy 21 by multiplexed maternal plasma DNA sequencing: large scale validity study", BMJ, vol. 342, no. jan11 1, 11 January 2011 (2011-01-11), pages c7401-c7401, XP055024134, ISSN: 0959-8138, DOI: 10.1136/bmj.c7401

## Description

### FIELD OF THE INVENTION

The present invention relates to a method for verifying the integrity of biological samples subjected to multistep bioassays that comprise massively parallel sequencing of the genomic nucleic acids of the biological samples.

### BACKGROUND OF THE INVENTION

Current sequencing technologies allow for the simultaneous analysis of many biological samples that can be assayed for a variety of determinations. For example, sequencing information relating to the genomic sequences in a sample can be used to determine the presence or absence of an aneuploidy, to diagnose disease or risk of disease, to identify associations between a phenotype and a genetic region, for paternity testing, and for forensic purposes.

It is important for most applications that each sample be properly identified as to source of origin and tracked during subsequent preparation and sequencing. For example, in a clinical setting, large numbers of samples are collected and processed, and information about sample donors must be maintained throughout the processes of sample preparation, sequencing, data collection and analysis to facilitate subsequent diagnoses. A single laboratory may service many clients, each client in turn requesting completion of multiple projects. Mishandling or sample misidentification mistakes could be of great harm when samples are used in the diagnosis of medical disorders *e*.*g*. prenatal diagnoses of chromosomal abnormalities, diagnoses of various disease states, and determinations of drug responses.

There is a need for a method suitable for verifying that sequencing information obtained by massively parallel sequencing of single or multiplexed samples corresponds to the originating biological source samples to ascertain the exclusion of accidental misidentification during multistep sample processing that is needed to provide nucleic acid preparations suitable for use in sequencing assays.

The present invention provides a reliable method that is applicable to sequencing assays practiced in the field of medicine, noninvasive diagnostics *e*.*g*. prenatal diagnostics.

WO 98/14275 discusses a method for validating the origin of a sample analysed by single-plex sequencing. WO 96/19586 considers a method for providing internal identification to a sample containing oligonucleotides. Multiplexed maternal plasma DNA sequencing whereby DNA from two or eight maternal plasma samples were co-sequenced using indexing sequences has been shown in Chiu et al., BMJ, vol. 342, pages c7401-c7401, (2011).

### SUMMARY OF THE INVENTION

The present invention relates to a method for verifying the integrity of biological samples subjected to multistep bioassays that comprise massively parallel sequencing of the genomic nucleic acids of the biological samples. The integrity of the biological samples is verified using unique marker nucleic acids that are combined with the biological source sample, concomitantly sequencing the marker nucleic acids and the genomic nucleic acids of the biological source sample, and verifying that the sequence information of the marker nucleic acid corresponds to that of the marker nucleic acid added to the biological source sample.

The invention provides a method for verifying the integrity of a plurality of biological source samples comprising genomic nucleic acids and determining the presence or absence of at least one fetal chromosomal abnormality in each of said plurality of biological source samples, said method comprising: (a) combining a unique marker nucleic acid with each of said plurality of biological source samples, wherein said plurality of biological source samples are maternal blood or plasma samples that comprise fetal and maternal cell-free DNA, thereby obtaining a plurality of uniquely marked samples each comprising a unique mixture of genomic and marker nucleic acids; (b) incorporating distinct indexing sequences into said genomic and marker nucleic acids of each of said uniquely marked samples thereby providing a uniquely marked indexed mixture of indexed marker and indexed sample nucleic acids for each of said plurality of source samples; (c) massively parallel sequencing a combination of uniquely marked indexed mixtures of indexed nucleic acids by multiplex sequencing; and (d) determining a correspondence between the sequence of said indexed marker and the sequence of said indexed genomic nucleic acids obtained in step (c) for each of said uniquely marked indexed mixtures of nucleic acids in said combination and the sequence of said unique marker nucleic acid in each of said uniquely marked samples, thereby verifying the integrity of each of said plurality of biological source samples, and determining the presence or absence of at least one fetal chromosomal abnormality in each of said plurality of marked indexed samples.

In one embodiment the method verifies the integrity of a plurality of biological source plasma samples comprising fetal and maternal cfDNA according to steps comprising: (a) combining a unique marker nucleic acid with each of the plurality of biological source samples, thereby obtaining a plurality of uniquely marked samples each comprising a unique mixture of genomic and marker nucleic acids; (b) incorporating distinct indexing sequences into the genomic and marker nucleic acids of each of the uniquely marked samples thereby providing a uniquely marked indexed mixture of indexed marker and indexed sample nucleic acids for each of the plurality of source samples; (c) massively parallel sequencing a combination of uniquely marked indexed mixtures of indexed nucleic acids; and (d) determining a correspondence between the sequence of the indexed marker and the sequence of the indexed genomic nucleic acids obtained in step (c) for each of the uniquely marked indexed mixtures of nucleic acids in the combination and the sequence of the unique marker nucleic acid in each of the uniquely marked samples, thereby verifying the integrity of each of the plurality of biological source samples and the method further comprises determining the presence or absence of at least one chromosomal abnormality in each of the plurality of marked indexed samples. In some embodiments, the at least one chromosomal abnormality is chosen from a partial chromosomal aneuploidy, a complete chromosomal aneuploidy, and a polymorphism. In other embodiments, the at least one chromosomal abnormality is associated with a disorder. The massively parallel sequencing can be of clonally-amplified cfDNA molecules. Alternatively, the massively parallel sequencing can be of single cfDNA molecules. The massively parallel sequencing can be massively parallel sequencing-by-synthesis, which can be performed using reversible dye terminators, massively parallel sequencing-by-ligation, massively parallel prosequencing, and/or massively parallel direct nucleotide interrogation sequencing. The method can further comprise isolating the unique mixture of genomic and marker nucleic acids for each of the plurality of samples. The marker nucleic acids can be DNA or analogs thereof. In some embodiments, the marker nucleic acid is between about 100bp and 600bp.

### SUMMARY OF THE DISCLOSURE

Disclosed herein is a method that verifies the integrity of a plurality of biological source samples comprising genomic nucleic acids according to steps comprising: (a) combining a unique marker nucleic acid with each of the plurality of biological source samples, thereby obtaining a plurality of uniquely marked samples each comprising a unique mixture of genomic and marker nucleic acids; (b) incorporating distinct indexing sequences into the genomic and marker nucleic acids of each of the uniquely marked samples thereby providing a uniquely marked indexed mixture of indexed marker and indexed sample nucleic acids for each of the plurality of source samples; (c) massively parallel sequencing a combination of uniquely marked indexed mixtures of indexed nucleic acids; and (d) determining a correspondence between the sequence of the indexed marker and the sequence of the indexed genomic nucleic acids obtained in step (c) for each of the uniquely marked indexed mixtures of nucleic acids in the combination and the sequence of the unique marker nucleic acid in each of the uniquely marked samples, thereby verifying the integrity of each of the plurality of biological source samples. The method can further comprise isolating the unique mixture of genomic and marker nucleic acids for each of the plurality of samples. The genomic nucleic acids can be cellular DNA or cell-free DNA. In some embodiments, the genomic nucleic acids can be RNA. The marker nucleic acids can be DNA or analogs thereof. In some embodiments, the marker nucleic acid is between about 100bp and 600bp.

Also disclosed herein is a method that verifies the integrity of a plurality of biological source samples comprising genomic nucleic acids according to steps comprising: (a) combining a unique marker nucleic acid with each of the plurality of biological source samples, thereby obtaining a plurality of uniquely marked samples each comprising a unique mixture of genomic and marker nucleic acids; (b) incorporating distinct indexing sequences into the genomic and marker nucleic acids of each of the uniquely marked samples thereby providing a uniquely marked indexed mixture of indexed marker and indexed sample nucleic acids for each of the plurality of source samples; (c) massively parallel sequencing a combination of uniquely marked indexed mixtures of indexed nucleic acids; and (d) determining a correspondence between the sequence of the indexed marker and the sequence of the indexed genomic nucleic acids obtained in step (c) for each of the uniquely marked indexed mixtures of nucleic acids in the combination and the sequence of the unique marker nucleic acid in each of the uniquely marked samples, thereby verifying the integrity of each of the plurality of biological source samples. In some embodiments, at least one of the plurality of biological samples is a maternal sample comprising a mixture of fetal and maternal nucleic acids, and the method can further comprise determining the presence or absence of at least one chromosomal abnormality in each of the plurality of marked indexed samples. In some embodiments, the at least one chromosomal abnormality is chosen from a partial chromosomal aneuploidy, a complete chromosomal aneuploidy, and a polymorphism. In other embodiments, the at least one chromosomal abnormality is associated with a disorder. The genomic nucleic acids is cellular DNA or cell-free DNA. In some embodiments, the genomic nucleic acids can be RNA. The marker nucleic acids can be DNA or analogs thereof. In some embodiments, the marker nucleic acid is between about 100bp and 600bp. In some embodiments, the method can comprise isolating the unique mixture of genomic and marker nucleic acids.

Also disclosed herein is a method that verifies the integrity of a plurality of biological source samples comprising genomic nucleic acids according to steps comprising: (a) combining a unique marker nucleic acid with each of the plurality of biological source samples, thereby obtaining a plurality of uniquely marked samples each comprising a unique mixture of genomic and marker nucleic acids; (b) incorporating distinct indexing sequences into the genomic and marker nucleic acids of each of the uniquely marked samples thereby providing a uniquely marked indexed mixture of indexed marker and indexed sample nucleic acids for each of the plurality of source samples; (c) massively parallel sequencing a combination of uniquely marked indexed mixtures of indexed nucleic acids; and (d) determining a correspondence between the sequence of the indexed marker and the sequence of the indexed genomic nucleic acids obtained in step (c) for each of the uniquely marked indexed mixtures of nucleic acids in the combination and the sequence of the unique marker nucleic acid in each of the uniquely marked samples, thereby verifying the integrity of each of the plurality of biological source samples. The biological samples can each comprise a mixture of nucleic acids from two or more genomes. In some embodiments, at least one of the plurality of biological samples is a maternal sample comprising a mixture of fetal and maternal nucleic acids, and the method can further comprise determining the presence or absence of at least one chromosomal abnormality in each of the plurality of marked indexed samples. The genomic nucleic acids can be cellular DNA or cell-free DNA. In some embodiments, the genomic nucleic acids can be RNA. The marker nucleic acids is DNA or analogs thereof. In some embodiments, the marker nucleic acid is between about 100bp and 600bp. In some embodiments, the method can comprise isolating the unique mixture of genomic and marker nucleic acids.

Also disclosed herein is a method that verifies the integrity of a plurality of biological source samples comprising genomic nucleic acids according to steps comprising: (a) combining a unique marker nucleic acid with each of the plurality of biological source samples, thereby obtaining a plurality of uniquely marked samples each comprising a unique mixture of genomic and marker nucleic acids; (b) incorporating distinct indexing sequences into the genomic and marker nucleic acids of each of the uniquely marked samples thereby providing a uniquely marked indexed mixture of indexed marker and indexed sample nucleic acids for each of the plurality of source samples; (c) massively parallel sequencing a combination of uniquely marked indexed mixtures of indexed nucleic acids; and (d) determining a correspondence between the sequence of the indexed marker and the sequence of the indexed genomic nucleic acids obtained in step (c) for each of the uniquely marked indexed mixtures of nucleic acids in the combination and the sequence of the unique marker nucleic acid in each of the uniquely marked samples, thereby verifying the integrity of each of the plurality of biological source samples. The source sample can be a biological fluid sample *e.g.* a blood sample, a plasma sample or a purified genomic nucleic acid sample. The method can further comprise isolating the unique mixture of genomic and marker nucleic acids for each of the plurality of samples. The genomic nucleic acids can be cellular DNA or cell-free DNA. In some embodiments, the genomic nucleic acids can be RNA. The marker nucleic acids is DNA or analogs thereof. In some embodiments, the marker nucleic acid is between about 100bp and 600bp.

Also disclosed herein is a method that verifies the integrity of a plurality of biological source samples comprising genomic nucleic acids according to steps comprising: (a) combining a unique marker nucleic acid with each of the plurality of biological source samples, thereby obtaining a plurality of uniquely marked samples each comprising a unique mixture of genomic and marker nucleic acids; (b) incorporating distinct indexing sequences into the genomic and marker nucleic acids of each of the uniquely marked samples thereby providing a uniquely marked indexed mixture of indexed marker and indexed sample nucleic acids for each of the plurality of source samples; (c) massively parallel sequencing a combination of uniquely marked indexed mixtures of indexed nucleic acids; and (d) determining a correspondence between the sequence of the indexed marker and the sequence of the indexed genomic nucleic acids obtained in step (c) for each of the uniquely marked indexed mixtures of nucleic acids in the combination and the sequence of the unique marker nucleic acid in each of the uniquely marked samples, thereby verifying the integrity of each of the plurality of biological source samples. The massively parallel sequencing can be of clonally-amplified cfDNA molecules. Alternatively, the massively parallel sequencing can be of single cfDNA molecules. The massively parallel sequencing can be massively parallel sequencing-by-synthesis, which can be performed using reversible dye terminators, massively parallel sequencing-by-ligation, massively parallel prosequencing, and/or massively parallel direct nucleotide interrogation sequencing. The method can further comprise isolating the unique mixture of genomic and marker nucleic acids for each of the plurality of samples. The genomic nucleic acid is cellular DNA or cell-free DNA. In some embodiments, the genomic nucleic acids can be RNA. The marker nucleic acids can be DNA or analogs thereof. In some embodiments, the marker nucleic acid is between about 100bp and 600bp.

Also disclosed herein is a method for verifying the integrity of a plurality of samples can be applied to verifying the integrity of a single biological source sample comprising genomic nucleic acids according to steps comprising: (a) combining unique marker nucleic acids with the biological source sample, thereby obtaining a marked sample comprising a mixture of genomic and marker nucleic acids; (b) massively parallel sequencing the mixture of nucleic acids; and (c) determining a correspondence between the sequence of the marker nucleic acid obtained in step (b) with the sequence of the marker nucleic acid added to the source sample, thereby verifying the integrity of the biological source sample. The genomic nucleic acids can be cfDNA. The genomic and marker nucleic acids can comprise identical indexing tags. The source sample can be a blood sample, a plasma sample, or a purified genomic nucleic acid sample. The marker nucleic acids can be DNA or analogs thereof. In some embodiments, the marker nucleic acid is between about 100bp and 600bp. The massively parallel sequencing can be of clonally-amplified cfDNA molecules. Alternatively, the massively parallel sequencing can be of single cfDNA molecules. The massively parallel sequencing can be massively parallel sequencing-by-synthesis, which can be performed using reversible dye terminators, massively parallel sequencing-by-ligation, massively parallel prosequencing, and/or massively parallel direct nucleotide interrogation sequencing.

Also disclosed herein is a kit comprising unique marker nucleic acids for verifying the integrity of each of a plurality of source samples in a bioassay comprising a massively parallel sequencing step. The kit can further comprise a set of indexing nucleic acid sequences.

### BRIEF DESCRIPTION OF THE DRAWINGS

The novel features of the invention are set forth with particularity in the appended claims. A better understanding of the features and advantages of the present invention will be obtained by reference to the following detailed description that sets forth illustrative embodiments, in which the principles of the invention are utilized, and the accompanying drawings of which:
**Figure 1** illustrates a flowchart of an embodiment **100** of the method for verifying the integrity of a sample that is subjected to a multistep singleplex sequencing bioassay.
**Figure 2** illustrates a flowchart of an embodiment **200** of the method for verifying the integrity of a plurality of samples that are subjected to a multistep multiplex sequencing bioassay.

### DETAILED DESCRIPTION

The present invention relates to a method for verifying the integrity of biological samples subjected to multistep bioassays that comprise massively parallel sequencing of the genomic nucleic acids of the biological samples. The integrity of the biological samples is verified using unique marker nucleic acids that are combined with the biological source sample, concomitantly sequencing the marker nucleic acids and the genomic nucleic acids of the biological source sample, and verifying that the sequence information of the marker nucleic acid corresponds to that of the marker nucleic acid added to the biological source sample.

Unless otherwise indicated, the practice of the present invention involves conventional techniques commonly used in molecular biology, microbiology, protein purification, protein engineering, protein and DNA sequencing, and recombinant DNA fields, which are within the skill of the art. Such techniques are known to those of skill in the art and are described in numerous texts and reference works (See *e*.*g*., Sambrook et al., "Molecular Cloning: A Laboratory Manual", Second Edition (Cold Spring Harbor), [1989]); and Ausubel et al., "Current Protocols in Molecular Biology" [1987]).

Numeric ranges are inclusive of the numbers defining the range. It is intended that every maximum numerical limitation given throughout this specification includes every lower numerical limitation, as if such lower numerical limitations were expressly written herein. Every minimum numerical limitation given throughout this specification will include every higher numerical limitation, as if such higher numerical limitations were expressly written herein. Every numerical range given throughout this specification will include every narrower numerical range that falls within such broader numerical range, as if such narrower numerical ranges were all expressly written herein.

The headings provided herein are not limitations of the various aspects or embodiments of the invention which can be had by reference to the Specification as a whole. Accordingly, as indicated above, the terms defined immediately below are more fully defined by reference to the specification as a whole.

Unless defined otherwise herein, all technical and scientific terms used herein have the same meaning as commonly understood by one of ordinary skill in the art to which this invention belongs. Various scientific dictionaries that include the terms included herein are well known and available to those in the art. Although any methods and materials similar or equivalent to those described herein find use in the practice or testing of the present invention, some preferred methods and materials are described. Accordingly, the terms defined immediately below are more fully described by reference to the Specification as a whole. It is to be understood that this invention is not limited to the particular methodology, protocols, and reagents described, as these may vary, depending upon the context they are used by those of skill in the art.

### Definitions

As used herein, the singular terms "a", "an," and "the" include the plural reference unless the context clearly indicates otherwise. Unless otherwise indicated, nucleic acids are written left to right in 5' to 3' orientation and amino acid sequences are written left to right in amino to carboxy orientation, respectively.

The term "sequencing bioassay" herein refers to a multistep bioassay that includes massively parallel sequencing of the sample nucleic acids *e.g.* cfDNA. Multistep bioassays can comprise one or more of the steps of sample collection, sample fractionation, nucleic acid purification, and the requisite nucleic acid modification steps for the preparation of sequencing libraries.

The term "Next Generation Sequencing (NGS)" herein refers to sequencing technologies that allow for massively parallel sequencing of clonally amplified and of single nucleic acid molecules.

The term "biological source sample" herein refers to a biological sample comprising genomic nucleic acids to which marker molecules are added. A biological source sample comprising marker nucleic acids is herein referred to as a "marked sample".

The term "indexing sequences" herein refers to distinct polynucleotide sequences that can be incorporated into marker and genomic nucleic acids during sequencing library preparation for multiplex sequencing of pooled libraries.

The term "genomic nucleic acids" herein refers to nucleic acids of biological samples *e.g*. deoxyribose nucleic acid (DNA) and ribonucleic acid (RNA).

The terms "marker nucleic acid" and "marker molecules" are used interchangeably to refer to polynucleotides that are used to track biological samples through multistep bioassays that comprise a massively parallel sequencing step. Marker nucleic acids can be deoxyribonucleic acids, ribonucleic acids, or analogs thereof. Marker molecules can have genomic or antigenomic sequences.

The terms "antigenomic polynucleotide" and "antigenomic sequence" are used herein interchangeably to refer to a polynucleotide having a sequence that is absent from the genome of the biological sample. Antigenomic sequences are used in bioassays of biological samples that comprise nucleic acid sequencing of the sample's nucleic acids. Genomic and antigenomic sequences can be used in assays of biological and non-biological samples that do not comprise sequencing of the sample's nucleic acids.

The term "marked sample" herein refers to a biological sample comprising genomic nucleic acids and marker nucleic acids. Different samples are marked with unique marker nucleic acids.

The term "purified" herein refers to material (e.g., an isolated polynucleotide) that is in a relatively pure state, *e.g.,* at least about 80% pure, at least about 85% pure, at least about 90% pure, at least about 95% pure, at least about 98% pure, or even at least about 99% pure.

The terms "extracted", "recovered," "isolated," and "separated," herein refer to a compound, protein, cell, nucleic acid or amino acid that is removed from at least one component with which it is naturally associated and found in nature.

The term "substantially cell free" herein encompasses preparations of the desired sample from which components that are normally associated with it are removed. For example, a plasma sample is rendered substantially cell free by removing blood cells *e.g.* red cells, which are normally associated with it.

The term "plurality" when used in reference to biological samples herein refers to two or more biological samples, which can be obtained for example, from two or more different subjects, or from one subj ect.

The term "unique" when used in reference to a marker nucleic acid herein refers to a marker nucleic acid having a sequence that is uniquely associated with a biological sample.

The term "determining a correspondence" herein refers to determining whether the sequence of the marker nucleic acid obtained from massively parallel sequencing is the sequence of the marker nucleic acid used to mark source sample. Similarly, "determining a correspondence" herein refers to determining whether the sequence of each of the unique marker nucleic acid obtained by massively parallel sequencing of a combination of mixtures of genomic and marker nucleic acids from different biological source samples corresponds to the sequence of the unique marker nucleic acid that was combined with each of the uniquely marked indexed samples in the combination.

The phrase "verifying the integrity of a source sample" herein refers to establishing whether the sequencing information is assigned correctly to the corresponding source sample.

The terms "source sample" and "biological source sample" are used interchangeably to refer to the original biological sample from which genomic nucleic acids are isolated and subsequently sequenced in a multistep bioassay.

The term "fractionation" herein refers to a separation process in which a certain quantity of a mixture (solid, liquid, solute, suspension) is divided up in a number of smaller quantities (fractions) in which the composition changes according to a gradient. Fractions are collected based on differences in a specific property of the individual components. A common trait in fractionations is the need to find an optimum between the amount of fractions collected and the desired purity in each fraction. Fractionation makes it possible to isolate more than two components in a mixture in a single run.

The term "clonally amplified" when used in reference to nucleic acid molecules herein refers to ensembles of copies of identical nucleic acid molecules that have been multiplied for sequencing.

The terms "disorder" and "genetic disorders'" are used herein interchangeably to refer to conditions or diseases that are caused in whole or in part by alterations in genes or chromosomes. The alterations in genes or chromosomes can be inherited, or can be the result of external factors such as infectious diseases. Disorders encompass single gene disorders including autosomal dominant, autosomal recessive, X-linked dominant, X-linked recessive, Y-linked, and polygenic disorders.

The term "maternal sample" herein refers to a biological sample obtained from a pregnant subject and that comprises a mixture of fetal and maternal nucleic acids *e.g*. cfDNA.

The terms "polymorphic target nucleic acid", "polymorphic sequence", "polymorphic target nucleic acid sequence" and "polymorphic nucleic acid" are used interchangeably herein to refer to a nucleic acid sequence *e.g.* a DNA sequence that comprises one or more polymorphic sites.

As used herein, the term "fetal fraction" is used interchangeably with "fraction of fetal nucleic acid", which refers to the fraction of fetal nucleic acid in a sample comprising fetal and maternal nucleic acid. Similarly, the term "minor fraction" or "minor component" herein refers to the lesser fraction of the total genetic material that is present in a sample containing genetic material derived from separate sources *e.g.* individuals.

The term "multiplex sequencing" herein refers to the sequencing of a mixture of pooled nucleic acids derived from two or more samples in a single lane of a flow cell or slide of a sequencer. Multiplex sequencing improves the productivity by reducing time and reagent use. Multiplex sequencing requires that samples be identifiable by incorporating a distinct index sequence to allow for appropriate analysis of sequencing information.

The term "singleplex sequencing" herein refers to the sequencing of nucleic acids derived from no more than one biological source sample in single lane of a flow cell or slide of a sequencer.

The term "pathogen" herein refers to a biological agent that can disrupt the normal physiology of its host, possibly causing a clinical condition.

The term "copy number variation (CNV)" herein refers to variation in the number of copies of a nucleic acid sequence that is 1 kb or larger present in a test sample in comparison with the copy number of the nucleic acid sequence present in a qualified sample *i*.*e*. normal sample. Copy number variations include deletions, including microdeletions, insertions, including microinsertions, duplications, multiplications, inversions, translocations and complex multi-site variants. CNV encompass complete chromosomal aneuploidies and partial aneuploidies.

The terms "polynucleotide" and "nucleic acid" are used interchangeably to refer to deoxyribonucleotides, ribonucleotides, or analogs thereof.

The terms "genomic molecule" and "genomic nucleic acid" are used interchangeably herein to refer to genomic nucleic acids, which can be cellular or cell-free nucleic acids.

The term "combination" when used in reference to sequencing "uniquely marked indexed mixtures of indexed nucleic acids" herein refers to multiplex sequencing of a plurality of mixtures of uniquely indexed mixtures of marker and genomic nucleic acids obtained from a corresponding plurality of biological source samples.

The term "chromosomal abnormality" herein refers to a genetic abnormality including but not limited to complete chromosomal aneuploidies, partial chromosomal aneuploidies, and polymorphisms.

The term "polymorphism" herein refers to a sequence variation within different alleles of the same genomic sequence. A sequence that contains a polymorphism is considered "polymorphic sequence". Detection of one or more polymorphisms allows differentiation of different alleles of a single genomic sequence or between two or more individuals. As used herein, the term "polymorphic marker" or "polymorphic sequence" refers to segments of genomic DNA that exhibit heritable variation in a DNA sequence between individuals. Such markers include, but are not limited to, single nucleotide polymorphisms (SNPs), tandem SNPs, restriction fragment length polymorphisms (RFLPs), short tandem repeats, such as di-, tri- or tetra-nucleotide repeats (STRs), and the like.

The term "complete chromosomal aneuploidy" herein refers to an imbalance of genetic material caused by a loss or gain of a whole chromosome, and includes germline aneuploidy and mosaic aneuploidy. Examples of complete chromosomal aneuploidies include trisomies, monosomies, tetrasomies and other polysomies.

The terms "partial aneuploidy" and "partial chromosomal aneuploidy" herein refer to an imbalance of genetic material caused by a loss or gain of part of a chromosome *e.g*. partial monosomy and partial trisomy, and encompasses imbalances resulting from translocations, deletions and insertions.

The term "disorder" herein refers to a medical condition that includes all diseases, but can include injuries and normal health situations, such as pregnancy, that might affect a person's health, benefit from medical assistance, or have implications for medical treatments.

The term "direct nucleotide interrogation sequencing" herein refers to single-molecule sequencing technology whereby a single nucleic acid molecule is sequenced directly as it passes through a detector. Nanopore sequencing is an example of direct nucleotide interrogation sequencing, whereby the sequencing process directly detects the bases of a nucleic acid strand as the strand passes through a nanopore.

### Detailed Description

The present invention relates to a method for verifying the integrity of biological source samples subjected to multistep bioassays that comprise massively parallel sequencing of the sample genomic nucleic acids. The integrity of the biological source samples is verified by combining a unique marker molecule of known sequence with the biological source sample, processing the marked sample to obtain a mixture of nucleic acids derived from the biological source sample and the marker molecule, which are sequenced concomitantly with the genomic nucleic acids of the biological source sample. Disclosed herein are methods that provide verification of individual samples in single- and multiplex massively parallel sequencing assays. The method disclosed is applicable to bioassays that comprise either singleplex or multiplex sequencing using sequencing technologies that may or may not require preparation of sequencing libraries. The method is particularly useful in methods of sample analysis that comprise massively parallel sequencing of sample nucleic acids that is performed in a multiplex fashion.

### Samples

The source sample comprising genomic nucleic acids to which the method described herein is applied is a biological sample such as a tissue sample, a biological fluid sample, or a cell sample, and processed fractions thereof. A biological fluid sample includes, as non-limiting examples, blood, plasma, serum, sweat, tears, sputum, urine, sputum, ear flow, lymph, interstitial fluid, saliva, cerebrospinal fluid, ravages, bone marrow suspension, vaginal flow, transcervical lavage, brain fluid, ascites, milk, secretions of the respiratory, intestinal and genitourinary tracts, amniotic fluid and leukophoresis samples. In some embodiments, the source sample is a sample that is easily obtainable by non-invasive procedures *e.g*. blood, plasma, serum, sweat, tears, sputum, urine, sputum, ear flow, and saliva. Preferably, the biological sample is a peripheral blood sample, or the plasma and serum fractions. In other embodiments, the biological sample is a swab or smear, a biopsy specimen, or a cell culture. In another embodiment, the sample is a mixture of two or more biological samples *e.g.* a biological sample can comprise two or more of a biological fluid sample, a tissue sample, and a cell culture sample. As used herein, the terms "blood," "plasma" and "serum" expressly encompass fractions or processed portions thereof. Similarly, where a sample is taken from a biopsy, swab, smear, etc., the "sample" expressly encompasses a processed fraction or portion derived from the biopsy, swab, smear, etc.

In some embodiments, samples can be obtained from sources, including, but not limited to, samples from different individuals, different developmental stages of the same or different individuals, different diseased individuals (e.g., individuals with cancer or suspected of having a genetic disorder), normal individuals, samples obtained at different stages of a disease in an individual, samples obtained from an individual subjected to different treatments for a disease, samples from individuals subjected to different environmental factors, or individuals with predisposition to a pathology, individuals with exposure to a pathogen such as an infectious disease agent (*e.g.,* HIV), and individuals who are recipients of donor cells, tissues and/or organs. In some embodiments, the sample is a sample comprising a mixture of different source samples derived from the same or different subjects. For example, a sample can comprise a mixture of cells derived from two or more individuals, as is often found at crime scenes. In one embodiment, the sample is a maternal sample that is obtained from a pregnant female, for example a pregnant woman. In this instance, the sample can be analyzed using the methods described herein to provide a prenatal diagnosis of potential fetal disorders. Unless otherwise specified, a maternal sample comprises a mixture of fetal and maternal DNA *e.g*. cfDNA. In some embodiments, the maternal sample is a biological fluid sample *e.g*. blood sample. In other embodiments, the maternal sample is a purified cfDNA sample.

A source sample can be an unprocessed biological sample *e.g.* a whole blood sample. A source sample can be a partially processed biological sample *e.g.* a blood sample that has been fractionated to provide a substantially cell-free plasma fraction. A source sample can be a biological sample containing purified nucleic acids *e.g.* a sample of purified cfDNA derived from an essentially cell-free plasma sample. Processing of the samples can include freezing samples *e.g.* tissue biopsy samples, fixing samples *e.g.* formalin-fixing, and embedding samples *e.g.* paraffin-embedding. Partial processing of samples include sample fractionation *e.g.* obtaining plasma fractions from blood samples, and other processing steps required for analyses of samples collected during routine clinical work, in the context of clinical trials, and/or scientific research. Additional processing steps can include steps for isolating and purifying sample nucleic acids. Further processing of purified samples includes for example, steps for the requisite modification of sample nucleic acids in preparation for sequencing. Preferably, the source sample is an unprocessed or a partially processed sample.

Samples can also be obtained from in vitro cultured tissues, cells, or other polynucleotide-containing sources. The cultured samples can be taken from sources including, but not limited to cultures (*e.g.,* tissue or cells) maintained in different media and conditions (*e.g.,* pH, pressure, or temperature), cultures *(e.g.,* tissue or cells) maintained for different periods of length, cultures (*e.g*., tissue or cells) treated with different factors or reagents (*e.g.,* a drug candidate, or a modulator), or cultures of different types of tissue or cells.

Biological source samples can be obtained from a variety of subjects including but not limited to human beings, and other organisms including mammals, plants, bacteria, or cells from said subjects.

Biological source samples are each combined with a unique marker nucleic acid which is used to verify that the sequencing information obtained for the sample nucleic acids corresponds to the source sample, thereby verifying the integrity of the source sample.

### Genomic nucleic acids

Verification of the integrity of the samples relies on sequencing mixtures of sample genomic nucleic acids *e.g*. cfDNA, and accompanying marker nucleic acids. Genomic nucleic acids include DNA and RNA, which can be cellular or cell-free. Preferably, genomic nucleic acids are cellular and/or cfDNA. In some embodiments, the genomic nucleic acid of the sample is cellular DNA, which can be derived from whole cells by manually or mechanically extracting the genomic DNA from whole cells of the same or of differing genetic compositions. Cellular DNA can be derived for example, from whole cells of the same genetic composition derived from one subject, from a mixture of whole cells of different subjects, or from a mixture of whole cells that differ in genetic composition that are derived from one subject. Methods for extracting genomic DNA from whole cells are known in the art, and differ depending upon the nature of the source. In some embodiments, it can be advantageous to fragment the cellular genomic DNA. Fragmentation can be random, or it can be specific, as achieved, for example, using restriction endonuclease digestion. Methods for random fragmentation are well known in the art, and include, for example, limited DNAse digestion, alkali treatment, and physical shearing. In some embodiments, sample nucleic acids are obtained as cellular genomic DNA, which is subjected to fragmentation into fragments of approximately 500 or more base pairs, which can be sequenced by next generation sequencing (NGS).

In some embodiments, cellular genomic DNA is obtained to identify chromosomal aneuploidies and/or polymorphisms of a sample comprising a single genome. For example, cellular genomic DNA can be obtained from a sample that contains only cells of a pregnant female *i.e.* the sample is free of fetal genomic sequences. Identification of chromosomal aneuploidies and/or polymorphisms from a single genome *e.g*. maternal only genome, can be used in a comparison with chromosomal aneuploidies and/or polymorphisms identified in a mixture of fetal and maternal genomes present in a maternal sample *e.g*. maternal plasma sample, to identify the fetal chromosomal aneuploidies and/or polymorphisms. Similarly, cellular genomic DNA can be obtained from a patient *e.g*. a cancer patient, at different stages of treatment to assess the efficacy of the therapeutic regimen by analyzing possible changes in chromosomal aneuploidies and/or polymorphisms in the sample DNA.

In some embodiments, it is advantageous to obtain cell-free nucleic acids *e.g*. cell-free DNA (cfDNA). Cell-free nucleic acids, including cell-free DNA, can be obtained by various methods known in the art from biological samples including but not limited to plasma, serum and urine (Fan et al., Proc Natl Acad Sci 105:16266-16271 [2008]; Koide et al., Prenatal Diagnosis 25:604-607 [2005]; Chen et al., Nature Med. 2: 1033-1035 [1996]; Lo et al., Lancet 350: 485-487 [1997]; Botezatu et al., Clin Chem. 46: 1078-1084, 2000; and Su et al., J Mol. Diagn. 6: 101-107 [2004]). To separate cfDNA from cells, fractionation, centrifugation (*e.g.,* density gradient centrifugation), DNA-specific precipitation, or high-throughput cell sorting and/or separation methods can be used. Commercially available kits for manual and automated separation of cfDNA are available (Roche Diagnostics, Indianapolis, IN, Qiagen, Valencia, CA, Macherey-Nagel, Duren, DE). Biological samples comprising cfDNA have been used in assays to determine the presence or absence of chromosomal abnormalities *e.g*. trisomy 21, by sequencing assays that can detect chromosomal aneuploidies and/or various polymorphisms.

The cfDNA present in the sample can be enriched specifically or non-specifically prior to preparing a sequencing library. Non-specific enrichment of sample DNA refers to the whole genome amplification of the genomic DNA fragments of the sample that can be used to increase the level of the sample DNA prior to preparing a cfDNA sequencing library. Non-specific enrichment can be the selective enrichment of one of the two genomes present in a sample that comprises more than one genome. For example, non-specific enrichment can be selective of the fetal genome in a maternal sample, which can be obtained by known methods to increase the relative proportion of fetal to maternal DNA in a sample. Alternatively, non-specific enrichment can be the non-selective amplification of both genomes present in the sample. For example, non-specific amplification can be of fetal and maternal DNA in a sample comprising a mixture of DNA from the fetal and maternal genomes. Methods for whole genome amplification are known in the art. Degenerate oligonucleotide-primed PCR (DOP), primer extension PCR technique (PEP) and multiple displacement amplification (MDA) are examples of whole genome amplification methods. In some embodiments, the sample comprising the mixture of cfDNA from different genomes is unenriched for cfDNA of the genomes present in the mixture. In other embodiments, the sample comprising the mixture of cfDNA from different genomes is non-specifically enriched for any one of the genomes present in the sample.

### Marker Nucleic Acids

Marker nucleic acids can be combined with biological source sample and subjected to multistep processes that include one or more of the steps of fractionating the biological source sample *e.g*. obtaining an essentially cell-free plasma fraction from a whole blood sample, purifying nucleic acids from a fractionated *e.g*. plasma, or unfractionated biological source sample *e.g.* a tissue sample, and sequencing. In some embodiments, sequencing comprises preparing a sequencing library. The sequence or combination of sequences of the marker molecules that are combined with a source sample is unique to the source sample. In some embodiments, the unique marker molecules in a sample all have the same sequence. In other embodiments, the unique marker molecules in a sample are a combination of two, three, four, five, six, seven, eight, nine, ten, fifteen, twenty, or more different sequences. In one embodiment, the integrity of a sample can be verified using a plurality of marker nucleic acid molecules having identical sequences. Alternatively, the identity of a sample can be verified using a plurality of marker nucleic acid molecules that have at least two, at least three, at least four, at least five, at least six, at least seven, at least eight, at least nine, at least ten, at least 15, at least 20, at least 30, at least 40, at least 50, or more different sequences. Verification of the integrity of the plurality of biological samples *i.e*. two or more biological samples, requires that each of the two or more samples be marked with marker nucleic acids that have sequences that are unique to each of the plurality of test sample that is being marked. For example, a first sample can be marked with a marker nucleic acid having sequence A, and a second sample can be marked with a marker nucleic acid having sequence B. Alternatively, a first sample can be marked with marker nucleic acid molecules all having sequence A, and a second sample can be marked with a mixture of sequences B and C, wherein sequences A, B and C are marker molecules having different sequences.

The marker nucleic acid can be added to the sample at any stage of sample preparation that occurs prior to library preparation and sequencing. In one embodiment, marker molecules can be combined with an unprocessed source sample. For example, the marker nucleic acid can be provided in a collection tube that is used to collect a blood sample. Alternatively, the marker nucleic acids can be added to the blood sample following the blood draw. In one embodiment, the marker nucleic acid is added to the vessel that is used to collect a biological fluid sample *e.g*. the marker nucleic acid is added to a blood collection tube that is used to collect a blood sample. In another embodiment, the marker nucleic acid is added to a fraction of the biological fluid sample. For example, the marker nucleic acid is added to the plasma and/or serum fraction of a blood sample *e.g.* a maternal plasma sample. In yet another embodiment, the marker molecules are added to a purified sample *e.g.* a sample of nucleic acids that have been purified from a biological sample. For example, the marker nucleic acid is added to a sample of purified maternal and fetal cfDNA. Similarly, the marker nucleic acids can be added to a biopsy specimen prior to processing the specimen. In some embodiments, the marker nucleic acids can be combined with a carrier that delivers the marker molecules into the cells of the biological sample. Cell-delivery carriers include pH-sensitive and cationic liposomes.

Marker molecules have antigenomic sequences, which are sequences that are absent from the genome of the biological source sample. In an exemplary embodiment, the marker molecules that are used to verify the integrity of a human biological source sample have sequences that are absent from the human genome. In an alternative embodiment, the marker molecules have sequences that are absent from the source sample and from any one or more other known genomes. For example, the marker molecules that are used to verify the integrity of a human biological source sample have sequences that are absent from the human genome and from the mouse genome. The alternative allows for verifying the integrity of a test sample that comprises two or more genomes. For example, the integrity of a human cell-free DNA sample obtained from a subject affected by a pathogen *e.g.* a bacterium, can be verified using marker molecules having sequences that are absent from both the human genome and the genome of the affecting bacterium. Sequences of genomes of numerous pathogens *e.g*. bacteria, viruses, yeasts, fungi, protozoa etc., are publicly available on the world wide web at ncbi.nlm.nih.gov/genomes. In another embodiment, marker molecules are nucleic acids that have sequences that are absent from any known genome. The sequences of marker molecules can be randomly generated algorithmically.

The marker molecules can be naturally-occurring deoxyribonucleic acids (DNA), ribonucleic acids or artificial nucleic acid analogs (nucleic acid mimics) including peptide nucleic acids (PMA), morpholino nucleic acid, locked nucleic acids, glycol nucleic acids, and threose nucleic acids, which are distinguished from naturally-occurring DNA or RNA by changes to the backbone of the molecule or DNA mimics that do not have a phosphodiester backbone. The deoxyribonucleic acids can be from naturally-occurring genomes or can be generated in a laboratory through the use of enzymes or by solid phase chemical synthesis. Chemical methods can also be used to generate the DNA mimics that are not found in nature. Derivatives of DNA are that are available in which the phosphodiester linkage has been replaced but in which the deoxyribose is retained include but are not limited to DNA mimics having backbones formed by thioformacetal or a carboxamide linkage, which have been shown to be good structural DNA mimics. Other DNA mimics include morpholino derivatives and the peptide nucleic acids (PNA), which contain an N-(2-aminoethyl)glycine-based pseudopeptide backbone (Ann Rev Biophys Biomol Struct 24:167-183 [1995]). PNA is an extremely good structural mimic of DNA (or of ribonucleic acid [RNA]), and PNA oligomers are able to form very stable duplex structures with Watson-Crick complementary DNA and RNA (or PNA) oligomers, and they can also bind to targets in duplex DNA by helix invasion (Mol Biotechnol 26:233-248 [2004]. Another good structural mimic/analog of DNA analog that can be used as a marker molecule is phosphorothioate DNA in which one of the non-bridging oxygens is replaced by a sulfur. This modification reduces the action of endo-and exonucleases2 including 5' to 3' and 3' to 5' DNA POL 1 exonuclease, nucleases S1 and P1, RNases, serum nucleases and snake venom phosphodiesterase.

The length of the marker molecules can be distinct or indistinct from that of the sample nucleic acids *i.e.* the length of the marker molecules can be similar to that of the sample genomic molecules, or it can be greater or smaller than that of the sample genomic molecules. The length of the marker molecules is measured by the number of nucleotide or nucleotide analog bases that constitute the marker molecule. Marker molecules having lengths that differ from those of the sample genomic molecules can be distinguished from source nucleic acids using separation methods known in the art. For example, differences in the length of the marker and sample nucleic acid molecules can be determined by electrophoretic separation *e.g*. capillary electrophoresis. Size differentiation can be advantageous for quantifying and assessing the quality of the marker and sample nucleic acids. Preferably, the marker nucleic acids are shorter than the genomic nucleic acids, and of sufficient length to exclude it from being mapped to the genome of the sample. For example, as a 30 base human sequence is needed to uniquely map it to a human genome, marker molecules used in sequencing bioassays of human samples should be at least 30 bp in length.

The choice of length of the marker molecule is determined primarily by the sequencing technology that is used to verify the integrity of a source sample. The length of the sample genomic nucleic acids being sequenced can also be considered. For example, some sequencing technologies employ clonal amplification of polynucleotides, which can require that the genomic polynucleotides that are to be clonally amplified be of a minimum length. For example, sequencing using the Illumina GAII sequence analyzer includes an in vitro clonal amplification by bridge PCR (also known as cluster amplification) of polynucleotides that have a minimum length of 110bp, to which adaptors are ligated to provide a nucleic acid of at least 200 bp and less than 600 bp that can be clonally amplified and sequenced. In some embodiments, the length of the adaptor-ligated marker molecule is between about 200bp and about 600bp, between about 250bp and 550bp, between about 300bp and 500bp, or between about 350 and 450. In other embodiments, the length of the adaptor-ligated marker molecule is about 200bp. For example, when sequencing fetal cfDNA that is present in a maternal sample, the length of the marker molecule can be chosen to be similar to that of fetal cfDNA molecules. Thus, in one embodiment, the length of the marker molecule used in an assay that comprises massively parallel sequencing of cfDNA in a maternal sample to determine the presence or absence of a fetal chromosomal aneuploidy, can be about 150 bp, about 160bp, 170 bp, about 180bp, about 190bp or about 200bp; preferably, the marker molecule is about 170 bp. Other sequencing approaches *e.g*. SOLiD sequencing, Polony Sequencing and 454 sequencing use emulsion PCR to clonally amplify DNA molecules for sequencing, and each technology dictates the minimum and the maximum length of the molecules that are to be amplified. The length of marker molecules to be sequenced as clonally amplified nucleic acids can be up to about 600bp. In some embodiments, the length of marker molecules to be sequenced can be greater than 600bp.

Single molecule sequencing technologies, which do not employ clonal amplification of molecules, and are capable of sequencing nucleic acids over a very broad range of template lengths, in most situations do not require that the molecules to be sequenced be of any specific length. However, the yield of sequences per unit mass is dependent on the number of 3' end hydroxyl groups, and thus having relatively short templates for sequencing is more efficient than having long templates. If starting with nucleic acids longer than 1000 nt, it is generally advisable to shear the nucleic acids to an average length of 100 to 200 nt so that more sequence information can be generated from the same mass of nucleic acids. Thus, the length of the marker molecule can range from tens of bases to thousands of bases. The length of marker molecules used for single molecule sequencing can be up to about 25bp, up to about 50bp, up to about 75bp, up to about 100bp, up to about 200bp, up to about 300bp, up to about 400bp, up to about 500bp, up to about 600bp, up to about 700bp, up to about 800 bp, up to about 900bp, up to about 1000bp, or more in length.

The length chosen for a marker molecule is also determined by the length of the genomic nucleic acid that is being sequenced. For example, cfDNA circulates in the human bloodstream as genomic fragments of cellular genomic DNA. Fetal cfDNA molecules found in the plasma of pregnant women are generally shorter than maternal cfDNA molecules (Chan et al., Clin Chem 50:8892 [2004]). Size fractionation of circulating fetal DNA has confirmed that the average length of circulating fetal DNA fragments is <300 bp, while maternal DNA has been estimated to be between about 0.5 and 1 Kb (Li et al., Clin Chem, 50: 1002-1011 [2004]). These findings are consistent with those of Fan et al., who determined using NGS that fetal cfDNA is rarely >340bp (Fan et al., Clin Chem 56:1279-1286 [2010]). DNA isolated from urine with a standard silica-based method consists of two fractions, high molecular weight DNA, which originates from shed cells and low molecular weight (150-250 base pair) fraction of transrenal DNA (Tr-DNA) (Botezatu et al., Clin Chem. 46: 1078-1084, 2000; and Su et al., J Mol. Diagn. 6: 101-107, 2004). The application of newly developed technique for isolation of cell-free nucleic acids from body fluids to the isolation of transrenal nucleic acids has revealed the presence in urine of DNA and RNA fragments much shorter than 150 base pairs (U.S. Patent Application Publication No. 20080139801). In embodiments, wherein cfDNA is the genomic nucleic acid that is sequenced, marker molecules that are chosen can be up to about the length of the cfDNA. For example, the length of marker molecules used in maternal cfDNA samples to be sequenced as single nucleic acid molecules or as clonally amplified nucleic acids can be between about 100 bp and 600. In other embodiments, the sample genomic nucleic acids are fragments of larger molecules. For example, a sample genomic nucleic acid that is sequenced is fragmented cellular DNA. In embodiments, when fragmented cellular DNA is sequenced, the length of the marker molecules can be up to the length of the DNA fragments. In some embodiments, the length of the marker molecules is at least the minimum length required for mapping the sequence read uniquely to the appropriate reference genome. In other embodiments, the length of the marker molecule is the minimum length that is required to exclude the marker molecule from being mapped to the sample reference genome.

In addition, marker molecules can be used to verify samples that are not assayed by nucleic acid sequencing, and that can be verified by common biotechniques other than sequencing *e.g*. real-time PCR (see Example 6).

### Sequencing methods

Sequencing methods that can be used to verify the integrity of a source sample comprise Next Generation Sequencing technologies, which allow multiple samples to be sequenced individually as marker and genomic molecules (*i.e*. singleplex sequencing) or as pooled samples as indexed marker and indexed genomic molecules (*i.e.* multiplex sequencing) on a single sequencing run, and generate up to several hundred million reads of DNA sequences. Sequences of marker and genomic nucleic acids, and of indexed marker and indexed genomic nucleic acids can be determined using Next Generation Sequencing Technologies (NGS) in which clonally amplified DNA templates or single DNA molecules, respectively, are sequenced in a massively parallel fashion (*e.g*. as described in Volkerding et al. Clin Chem 55:641-658 [2009]; Metzker M Nature Rev 11:31-46 [2010]). NGS technologies are sometimes subclassified as First, Second and Third Generation Sequencing (Pareek and Smoczynski, J Appl Genetics 52:413-435 [2011]). In addition to high-throughput sequence information, NGS provide quantitative information, in that each sequence read can be a countable "sequence tag" representing an individual clonal DNA template or a single DNA molecule. The sequencing technologies of NGS include without limitation pyrosequencing, sequencing-by-synthesis with reversible dye terminators, sequencing by oligonucleotide probe ligation and ion semiconductor sequencing.

Some of the sequencing technologies are available commercially, such as the sequencing-by-hybridization platform from Affymetrix Inc. (Sunnyvale, CA) and the sequencing-by-synthesis platforms from 454 Life Sciences (Bradford, CT), Illumina/Solexa (Hayward, CA) and Helicos Biosciences (Cambridge, MA), and the sequencing-by-ligation platform from Applied Biosystems (Foster City, CA), as described below. In addition to the single molecule sequencing performed using sequencing-by-synthesis of Helicos Biosciences, other single molecule sequencing technologies include the SMRT™ technology of Pacific Biosciences, the Ion Torrent^{™} technology, and nanopore sequencing being developed for example, by Oxford Nanopore Technologies. While the automated Sanger method is considered as a 'first generation' technology, the method disclosed herein can be applied to bioassays that use Sanger sequencing, including automated Sanger sequencing. In addition, the method disclosed herein can be applied to bioassays that use nucleic acid imaging technologies *e.g*. atomic force microscopy (AFM) or transmission electron microscopy (TEM). Exemplary sequencing technologies are described below.

In one embodiment, the method can be applied to bioassays that use single molecule sequencing technology the Helicos True Single Molecule Sequencing (tSMS) technology (*e.g*. as described in Harris T.D. et al., Science 320:106-109 [2008]). In the tSMS technique, a DNA sample is cleaved into strands of approximately 100 to 200 nucleotides, and a polyA sequence is added to the 3' end of each DNA strand. Each strand is labeled by the addition of a fluorescently labeled adenosine nucleotide. The DNA strands are then hybridized to a flow cell, which contains millions of oligo-T capture sites that are immobilized to the flow cell surface. The templates can be at a density of about 100 million templates/cm². The flow cell is then loaded into an instrument, *e.g.,* HeliScope™ sequencer, and a laser illuminates the surface of the flow cell, revealing the position of each template. A CCD camera can map the position of the templates on the flow cell surface. The template fluorescent label is then cleaved and washed away. The sequencing reaction begins by introducing a DNA polymerase and a fluorescently labeled nucleotide. The oligo-T nucleic acid serves as a primer. The polymerase incorporates the labeled nucleotides to the primer in a template directed manner. The polymerase and unincorporated nucleotides are removed. The templates that have directed incorporation of the fluorescently labeled nucleotide are discerned by imaging the flow cell surface. After imaging, a cleavage step removes the fluorescent label, and the process is repeated with other fluorescently labeled nucleotides until the desired read length is achieved. Sequence information is collected with each nucleotide addition step. Whole genome sequencing by single molecule sequencing technologies excludes PCR-based amplification in the preparation of the sequencing libraries, and the directness of sample preparation allows for direct measurement of the sample, rather than measurement of copies of that sample.

In another embodiment, the method can be applied to bioassays that use 454 sequencing (Roche) (*e.g.* as described in Margulies, M. et al. Nature 437:376-380 [2005]). 454 sequencing involves two steps. In the first step, DNA is sheared into fragments of approximately 300-800 base pairs, and the fragments are blunt-ended. Oligonucleotide adaptors are then ligated to the ends of the fragments. The adaptors serve as primers for amplification and sequencing of the fragments. The fragments can be attached to DNA capture beads, *e.g*., streptavidin-coated beads using, *e.g.*, Adaptor B, which contains 5'-biotin tag. The fragments attached to the beads are PCR amplified within droplets of an oil-water emulsion. The result is multiple copies of clonally amplified DNA fragments on each bead. In the second step, the beads are captured in wells (pico-liter sized). Pyrosequencing is performed on each DNA fragment in parallel. Addition of one or more nucleotides generates a light signal that is recorded by a CCD camera in a sequencing instrument. The signal strength is proportional to the number of nucleotides incorporated. Pyrosequencing makes use of pyrophosphate (PPi) which is released upon nucleotide addition. PPi is converted to ATP by ATP sulfurylase in the presence of adenosine 5' phosphosulfate. Luciferase uses ATP to convert luciferin to oxyluciferin, and this reaction generates light that is discerned and analyzed.

In another embodiment, the DNA sequencing technology that is used in the method of the invention is the SOLiD™ technology (Applied Biosystems). In SOLiD™ sequencing-by-ligation, genomic DNA is sheared into fragments, and adaptors are attached to the 5' and 3' ends of the fragments to generate a fragment library. Alternatively, internal adaptors can be introduced by ligating adaptors to the 5' and 3' ends of the fragments, circularizing the fragments, digesting the circularized fragment to generate an internal adaptor, and attaching adaptors to the 5' and 3' ends of the resulting fragments to generate a mate-paired library. Next, clonal bead populations are prepared in microreactors containing beads, primers, template, and PCR components. Following PCR, the templates are denatured and beads are enriched to separate the beads with extended templates. Templates on the selected beads are subjected to a 3' modification that permits bonding to a glass slide. The sequence can be determined by sequential hybridization and ligation of partially random oligonucleotides with a central determined base (or pair of bases) that is identified by a specific fluorophore. After a color is recorded, the ligated oligonucleotide is cleaved and removed and the process is then repeated.

In another embodiment, the method can be applied to bioassays that use the single molecule, real-time (SMRT™) sequencing technology of Pacific Biosciences. In SMRT sequencing, the continuous incorporation of dye-labeled nucleotides is imaged during DNA synthesis. SMRT sequencing is an example of real-time sequencing, which involves imaging of the continuous incorporation of dye-labelled nucleotides during DNA sysnthesis. The techonology uases single DNA polymerase molecules that are attached to the bottom surface of individual zero-mode wavelength identifiers (ZMW identifiers) and that obtain sequence information while phospolinked nucleotides are being incorporated into the growing primer strand. A ZMW is a confinement structure which enables observation of incorporation of a single nucleotide by DNA polymerase against the background of fluorescent nucleotides that rapidly diffuse in an out of the ZMW (in microseconds). It takes several milliseconds to incorporate a nucleotide into a growing strand. During this time, the fluorescent label is excited and produces a fluorescent signal, and the fluorescent tag is cleaved off. Identification of the corresponding fluorescence of the dye indicates which base was incorporated. The process is repeated. Other real-time sequencing technologies that can be employed with the method include that of VisiGen and LI-COR Biosciences. VisiGen has engineered DNA polymerases with attached fluorescent that upon incorporation of their γ-labelled nucleotides, produce an enhanced signal by fluorescence resonance energy transfer; and LI-COR Biosciences uses dye-quencer nucleotides, which in their native state produce low signals owing to the presence of a quencher group attached to the base. The release and diffusion of the dye-labelled pyrophosphate analogue produces a fluorescent signal.

In another embodiment, the method can be applied to bioassays that use nanopore sequencing (*e.g*. as described in Soni GV and Meller A. Clin Chem 53: 1996-2001 [2007]). Nanopore sequencing DNA analysis techniques are being industrially developed by a number of companies, including Oxford Nanopore Technologies (Oxford, United Kingdom). Nanopore sequencing is a single-molecule sequencing technology whereby a single molecule of DNA is sequenced directly as it passes through a nanopore. Nanopore sequencing is an example of direct nucleotide interrogation sequencing, whereby the sequencing process directly detects the bases of a nucleic acid strand as the strand passes through a detector. Another example of direct nucleotide interrogation sequencing is that of Halcyon described below. A nanopore is a small hole, of the order of 1 nanometer in diameter. Immersion of a nanopore in a conducting fluid and application of a potential (voltage) across it results in a slight electrical current due to conduction of ions through the nanopore. The amount of current which flows is sensitive to the size and shape of the nanopore. As a DNA molecule passes through a nanopore, each nucleotide on the DNA molecule obstructs the nanopore to a different degree, changing the magnitude of the current through the nanopore in different degrees. Thus, this change in the current as the DNA molecule passes through the nanopore represents a reading of the DNA sequence.

In another embodiment, the method can be applied to bioassays that use the chemical-sensitive field effect transistor (chemFET) array (*e.g.,* as described in U.S. Patent Application Publication No. 20090026082). In one example of the technique, DNA molecules can be placed into reaction chambers, and the template molecules can be hybridized to a sequencing primer bound to a polymerase. Incorporation of one or more triphosphates into a new nucleic acid strand at the 3' end of the sequencing primer can be discerned by a change in current by a chemFET. An array can have multiple chemFET sensors. In another example, single nucleic acids can be attached to beads, and the nucleic acids can be amplified on the bead, and the individual beads can be transferred to individual reaction chambers on a chemFET array, with each chamber having a chemFET sensor, and the nucleic acids can be sequenced.

In another embodiment, the method can be applied to bioassays that use the Halcyon Molecular's method that uses transmission electron microscopy (TEM). The method, termed Individual Molecule Placement Rapid Nano Transfer (IMPRNT), comprises utilizing single atom resolution transmission electron microscope imaging of high-molecular weight (150kb or greater) DNA selectively labeled with heavy atom markers and arranging these molecules on ultra-thin films in ultra-dense (3nm strand-to-strand) parallel arrays with consistent base-to-base spacing. The electron microscope is used to image the molecules on the films to determine the position of the heavy atom markers and to extract base sequence information from the DNA. The method is further described in PCT patent publication WO 2009/046445. The method allows for sequencing complete human genomes in less than ten minutes.

In another embodiment, the DNA sequencing technology is the Ion Torrent single molecule sequencing, which pairs semiconductor technology with a simple sequencing chemistry to directly translate chemically encoded information (A, C, G, T) into digital information (0, 1) on a semiconductor chip. In nature, when a nucleotide is incorporated into a strand of DNA by a polymerase, a hydrogen ion is released as a byproduct. Ion Torrent uses a high-density array of micro-machined wells to perform this biochemical process in a massively parallel way. Each well holds a different DNA molecule. Beneath the wells is an ion-sensitive layer and beneath that an ion sensor. When a nucleotide, for example a C, is added to a DNA template and is then incorporated into a strand of DNA, a hydrogen ion will be released. The charge from that ion will change the pH of the solution, which can be identified by Ion Torrent's ion sensor. The sequencer-essentially the world's smallest solid-state pH meter-calls the base, going directly from chemical information to digital information. The Ion personal Genome Machine (PGM™) sequencer then sequentially floods the chip with one nucleotide after another. If the next nucleotide that floods the chip is not a match. No voltage change will be recorded and no base will be called. If there are two identical bases on the DNA strand, the voltage will be double, and the chip will record two identical bases called. Direct identification allows recordation of nucleotide incorporation in seconds.

In another embodiment, the present method can be applied to bioassays that uses massively parallel sequencing of millions of DNA fragments using Illumina's sequencing-by-synthesis and reversible terminator-based sequencing chemistry (*e.g*. as described in Bentley et al., Nature 6:53-59 [2009]). Template DNA can be genomic DNA *e.g*. cfDNA. In some embodiments, genomic DNA from isolated cells is used as the template, and it is fragmented into lengths of several hundred base pairs. In other embodiments, cfDNA is used as the template, and fragmentation is not required as cfDNA exists as short fragments. For example fetal cfDNA circulates in the bloodstream as fragments of <300 bp, and maternal cfDNA has been estimated to circulate as fragments of between about 0.5 and 1 Kb (Li et al., Clin Chem, 50: 1002-1011 [2004]). Illumina's sequencing technology relies on the attachment of fragmented genomic DNA to a planar, optically transparent surface on which oligonucleotide anchors are bound. Template DNA is end-repaired to generate 5'-phosphorylated blunt ends, and the polymerase activity of Klenow fragment is used to add a single A base to the 3' end of the blunt phosphorylated DNA fragments. This addition prepares the DNA fragments for ligation to oligonucleotide adapters, which have an overhang of a single T base at their 3' end to increase ligation efficiency. The adapter oligonucleotides are complementary to the flow-cell anchors. Under limiting-dilution conditions, adapter-modified, single-stranded template DNA is added to the flow cell and immobilized by hybridization to the anchors. Attached DNA fragments are extended and bridge amplified to create an ultra-high density sequencing flow cell with hundreds of millions of clusters, each containing∼1,000 copies of the same template. In one embodiment, the randomly fragmented genomic DNA *e.g*. cfDNA, is amplified using PCR before it is subjected to cluster amplification. Alternatively, an amplification-free genomic library preparation is used, and the randomly fragmented genomic DNA *e.g*. cfDNA is enriched using the cluster amplification alone (Kozarewa et al., Nature Methods 6:291-295 [2009]). The templates are sequenced using a robust four-color DNA sequencing-by-synthesis technology that employs reversible terminators with removable fluorescent dyes. High-sensitivity fluorescence identification is achieved using laser excitation and total internal reflection optics. Short sequence reads of about 20-40 bp *e.g.* 36 bp, are aligned against a repeat-masked reference genome and genetic differences are called using specially developed data analysis pipeline software. After completion of the first read, the templates can be regenerated in situ to enable a second read from the opposite end of the fragments. Thus, either single-end or paired end sequencing of the DNA fragments can be used. Partial sequencing of DNA fragments present in the sample is performed, and sequence tags comprising reads of predetermined length *e.g.* 36 bp, are mapped to a known reference genome. The mapped tags can be counted.

### Singleplex sequencing

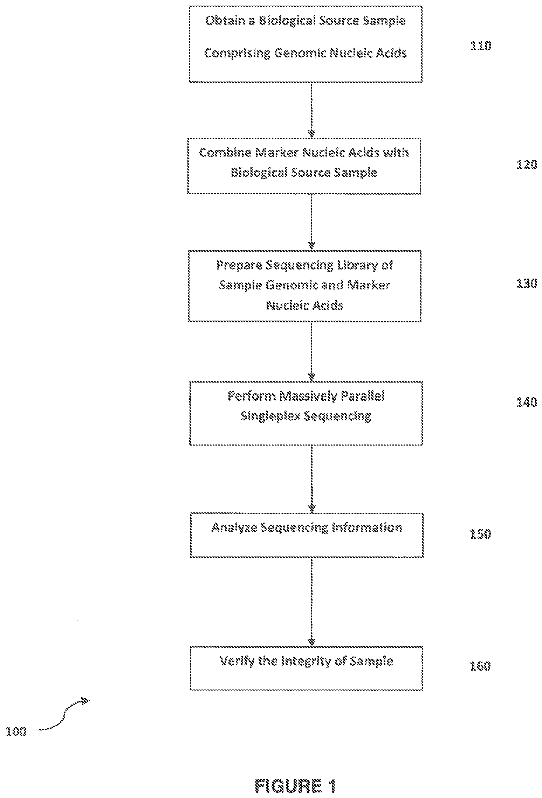

**Figure 1** illustrates a flow chart of an embodiment of the method whereby marker nucleic acids are combined with source sample nucleic acids of a single sample to assay for a genetic abnormality while determining the integrity of the biological source sample. In step **110,** a biological source sample comprising genomic nucleic acids is obtained. In step **120,** marker nucleic acids are combined with the biological source sample to provide a marked sample. A sequencing library of a mixture of clonally amplified source sample genomic and marker nucleic acids is prepared in step **130,** and the library is sequenced in a massively parallel fashion in step **140** to provide sequencing information pertaining to the source genomic and marker nucleic acids of the sample. Massively parallel sequencing methods provide sequencing information as sequence reads, which are mapped to one or more reference genomes to generate sequence tags that can be analyzed. In step **150,** all sequencing information is analyzed, and based on the sequencing information pertaining to the marker molecules, the integrity of the source sample is verified in step **160.** Verification of source sample integrity is accomplished by determining a correspondence between the sequencing information obtained for the maker molecule at step **150** and the known sequence of the marker molecule that was added to the original source sample at step **120.** The same process can be applied to multiple samples that are sequenced separately, with each sample comprising molecules having sequences unique to the sample *i.e.* one sample is marked with a unique marker molecule and it is sequenced separately from other samples in a flow cell or slide of a sequencer. If the integrity of the sample is verified, the sequencing information pertaining to the genomic nucleic acids of the sample can be analyzed to provide information *e.g*. about the status of the subject from which the source sample was obtained. For example, if the integrity of the sample is verified, the sequencing information pertaining to the genomic nucleic acids is analyzed to determine the presence or absence of a chromosomal abnormality. If the integrity of the sample is not verified, the sequencing information is disregarded.

The method depicted in **Figure 1** is also applicable to bioassays that comprise singleplex sequencing of single molecules *e.g*. tSMS by Helicos, SMRT by Pacific Biosciences, BASE by Oxford Nanopore, and other technologies such as that suggested by IBM, which do not require preparation of libraries.

### Multiplex sequencing

The large number of sequence reads that can be obtained per sequencing run permits the analysis of pooled samples *i.e.* multiplexing, which maximizes sequencing capacity and reduces workflow. For example, the massively parallel sequencing of 8 libraries performed using the 8 lane flow cell of the Illumina Genome Analyzer can be multiplexed to sequence two or more samples in each lane such that 16, 24, 32 etc. or more samples can be sequenced in a single run. Parallelizing sequencing for multiple samples *i.e.* multiplex sequencing, requires the incorporation of sample-specific index sequences, also known as barcodes, during the preparation of sequencing libraries. Sequencing indexes are distinct base sequences of about 5, about 10, about 15, about 20 about 25, or more bases that are added at the 3' end of the genomic and marker nucleic acid. The multiplexing system enables sequencing of hundreds of biological samples within a single sequencing run. The preparation of indexed sequencing libraries for sequencing of clonally amplified sequences can be performed by incorporating the index sequence into one of the PCR primers used for cluster amplification. Alternatively, the index sequence can be incorporated into the adaptor, which is ligated to the cfDNA prior to the PCR amplification. Indexed libraries for single molecule sequencing can be created by incorporating the index sequence at the 3' end of the marker and genomic molecule or 5' to the addition of a sequence needed for hybridization to the flow cell anchors *e.g*. addition of the polyA tail for single molecule sequencing using the tSMS. Sequencing of the uniquely marked indexed nucleic acids provides index sequence information that identifies samples in the pooled sample libraries, and sequence information of marker molecules correlates sequencing information of the genomic nucleic acids to the sample source. In embodiments wherein the multiple samples are sequenced individually *i.e*. singleplex sequencing, marker and genomic nucleic acid molecules of each sample need only be modified to contain the adaptor sequences as required by the sequencing platform and exclude the indexing sequences.

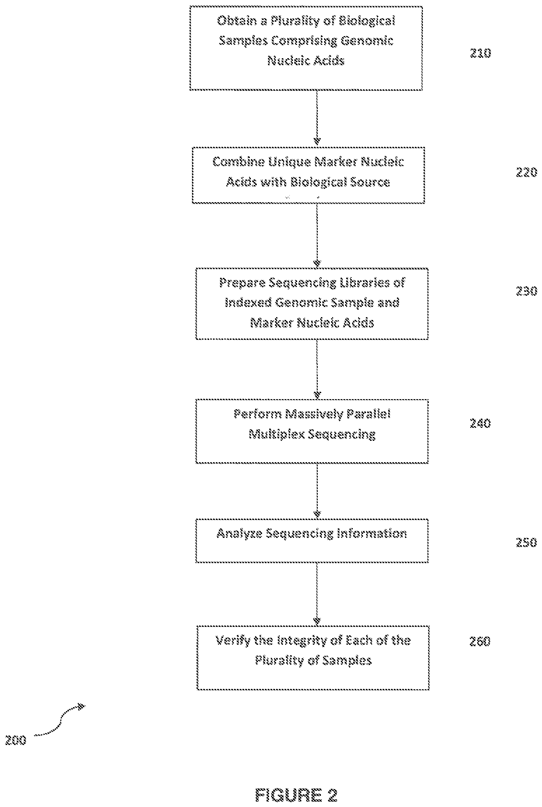

**Figure 2** provides a flowchart of an embodiment **200** of the method for verifying the integrity of samples that are subjected to a multistep multiplex sequencing bioassay *i.e*. nucleic acids from individual samples are combined and sequenced as a complex mixture. In step **210,** a plurality of biological source samples each comprising genomic nucleic acids is obtained. In step **220,** unique marker nucleic acids are combined with each of the biological source samples to provide a plurality of uniquely marked samples. A sequencing library of sample genomic and marker nucleic acids is prepared in step **230** for each of the uniquely marked samples. Library preparation of samples that are destined to undergo multiplexed sequencing comprises the incorporation of distinct indexing tags into the sample and marker nucleic acids of each of the uniquely marked samples to provide samples whose source nucleic acid sequences can be correlated with the corresponding marker nucleic acid sequences and identified in complex solutions. In embodiments of the method comprising marker molecules that can be enzymatically modified, *e.g*. DNA, indexing molecules can be incorporated at the 3' of the sample and marker molecules by ligating sequenceable adaptor sequences comprising the indexing sequences. In embodiments of the method comprising marker molecules that cannot be enzymatically modified, *e.g*. DNA analogs that do not have a phosphate backbone, indexing sequences are incorporated at the 3' of the analog marker molecules during synthesis. Sequencing libraries of two or more samples are pooled and loaded on the flow cell of the sequencer where they are sequenced in a massively parallel fashion in step **240.** In step **250,** all sequencing information is analyzed, and based on the sequencing information pertaining to the marker molecules; the integrity of the source sample is verified in step **260.** Verification of the integrity of each of the plurality of source samples is accomplished by first grouping sequence tags associated with identical index sequences to associate the genomic and marker sequences and distinguish sequences belonging to each of the libraries made from genomic molecules of a plurality of samples. Analysis of the grouped marker and genomic sequences is then performed to verify that the sequence obtained for the marker molecules corresponds to the known unique sequence added to the corresponding source sample. If the integrity of the sample is verified, the sequencing information pertaining to the genomic nucleic acids of the sample can be analyzed to provide genetic information about the subject from which the source sample was obtained. For example, if the integrity of the sample is verified, the sequencing information pertaining to the genomic nucleic acids is analyzed to determine the presence or absence of a chromosomal abnormality. The absence of a correspondence between the sequencing information and known sequence of the marker molecule is indicative of a sample mix-up, and the accompanying sequencing information pertaining to the genomic cfDNA molecules is disregarded.

### Analysis of sequencing information

NGS technologies provide sequence reads that vary in size from tens to hundreds of base pairs. In some embodiments of the method described herein, the sequence reads are about 20bp, about 25bp, about 30bp, about 35bp, about 40bp, about 45bp, about 50bp, about 55bp, about 60bp, about 65bp, about 70bp, about 75bp, about 80bp, about 85bp, about 90bp, about 95bp, about 100bp, about 110bp, about 120bp, about 130, about 140bp, about 150bp, about 200bp, about 250bp, about 300bp, about 350bp, about 400bp, about 450bp, or about 500bp. It is expected that technological advances will enable single-end reads of greater than 500bp enabling for reads of greater than about 1000bp when paired end reads of clonally amplified molecules are generated, and reads of>5000bp generated by single molecule sequencing. The massive quantity of sequence output is transferred by an analysis pipeline that transforms primary imaging output from the sequencer into strings of bases. A package of integrated algorithms performs the core primary data transformation steps: *e.g.* image analysis, intensity scoring, base calling, and alignment.

Sequencing of sample and marker molecules is performed, and sequence tags comprising reads of predetermined length *e.g.* 36 bp, are mapped to a known genomic sequences corresponding to the genome of the sample molecules, and to known synthetic sequences corresponding to the sequences of the marker molecules, respectively. Mapping of the sequence tags is achieved by comparing the sequence of the tag with the sequence of the reference genome to determine the chromosomal origin of the sequenced nucleic acid (*e.g.* cfDNA) molecule, and specific genetic sequence information is not needed. A number of computer algorithms are available for aligning sequences, including without limitation BLAST (Altschul et al., 1990), BLITZ (MPsrch) (Sturrock & Collins, 1993), FASTA (Person & Lipman, 1988), BOWTIE (Langmead et al., Genome Biology 10:R25.1-R25.10 [2009]), or ELAND (Illumina, Inc., San Diego, CA, USA). One or both ends of the clonally expanded copies of the plasma cfDNA molecules can be sequenced and processed by bioinformatic alignment analysis for the Illumina Genome Analyzer, which uses the Efficient Large-Scale Alignment of Nucleotide Databases (ELAND) software.

The mapped tags can be counted and/or assembled to compile a partial or entire genome of the sample *i.e.* whole genome sequencing. Only sequence reads that uniquely align to the reference genome are considered as sequence tags. The reference genome used for mapping sequencing information obtained for a human source sample can be for example, the human reference genome NCBI36/hg19 sequence, which is available on the world wide web at genome.ucsc.edu/cgi-bin/hgGateway?org=Human&db=hg19&hgsid=166260105). Other sources of public sequence information include GenBank, dbEST, dbSTS, EMBL (the European Molecular Biology Laboratory), and the DDBJ (the DNA Databank of Japan).

Verification of the integrity of samples that are sequenced individually *i.e.* singleplex sequencing, is determined by mapping sequence reads to a known genome and to a synthetic genome comprising the sequence of the marker molecules. Verification of the integrity of samples that are sequenced in a solution of combined mixtures of indexed sample and indexed marker molecules derived from two or more samples *i.e.* multiplex sequencing, is determined by first grouping sequence information of marker and genomic molecules by the index sequences, followed by mapping the sequence reads related to the index information to a known reference genome and to a synthetic genome comprising the sequence of the marker molecules.

### Applications

The method for verifying the integrity of a sample as described herein is applicable to any bioassay that includes and provides sequencing information of the genetic material of the sample. For example, the method can be applied to assays of whole-genome and candidate region resequencing, transcriptome analysis, small RNA discovery, methylation profiling, and genome-wide protein-nucleic acid interaction analysis. The method can be used in bioassays for determining chromosomal abnormalities including changes in copy number of complete and partial chromosomal sequences *i.e.* copy number variations, including deletions, including microdeletions, insertions, including microinsertions, duplications, multiplications, inversions, translocations and complex multi-site variants, and polymorphisms including but not limited to single nucleotide polymorphisms (SNPs), tandem SNPs, small-scale multi-base deletions or insertions, called IN-DELS (also called deletion insertion polymorphisms or DIPs), Multi-Nucleotide Polymorphisms (MNPs) Short Tandem Repeats (STRs), restriction fragment length polymorphism (RFLP).

The method can be used in bioassays with applications including but not limited to determinations of the presence or absence of chromosomal abnormalities indicative of a disease *e.g*. cancer, and/or the status of a disease, determinations of chromosomal abnormalities indicative of a genetic condition in a fetus *e.g*. trisomy 21, determinations of the presence or absence of nucleic acids of a pathogen *e.g.* virus, detection of chromosomal abnormalities associated with graft versus host disease (GVHD), and determinations of the contribution of individuals in forensic analyses.

In some embodiments, the method can be used to verify the integrity of a biological source sample that is obtained from a pregnant female *e.g.* a pregnant human, and is subjected to NGS for determining the presence or absence of a fetal chromosomal abnormality. In one embodiment, the method verifies the integrity of a plurality of biological source samples of which at least one is a maternal sample, by (a) combining a unique marker nucleic acid with each of the plurality of biological source samples, thereby obtaining a plurality of uniquely marked samples each comprising a unique mixture of genomic and marker nucleic acids; (b) incorporating distinct indexing sequences into the genomic and marker nucleic acids of each of said uniquely marked samples thereby providing a uniquely marked indexed mixture of indexed marker and indexed sample nucleic acids for each of the plurality of source samples; (c) massively parallel sequencing a combination of uniquely marked indexed mixtures of indexed nucleic acids; and (d) determining a correspondence between the sequence of the indexed marker and the sequence of indexed genomic nucleic acids obtained in step (d) for each of the uniquely marked indexed mixtures of nucleic acids in the combination and the sequence of the unique marker nucleic acid in each of the uniquely marked samples, thereby verifying the integrity of each of the plurality of biological source samples. The maternal sample can be any biological sample that comprises fetal and maternal nucleic acids *e.g*. cfDNA. Preferably, the maternal sample is a sample that is obtained by non-invasive procedures. In some embodiments, the maternal source sample is a peripheral blood sample. In other embodiments, the maternal source sample is a plasma sample. Sequencing of fetal and maternal nucleic acids can be achieved by any one of the massively parallel sequencing methods, and determining the presence or absence of fetal chromosomal abnormalities can be performed according to exemplary methods disclosed in US Patents Nos. 7,888,017, 8,008,018, and 8,137,912, U.S. Patent Application Publication Nos. US 2007/0202525A1; US2010/0112575A1, US 2009/0087847A1; US2009/0029377A1; US 2008/0220422A1; US2008/0138809A1, US2011/0201507, US 2011/0245085, US2011/0230358, US2011/0177517, and Fan and Quake 2010 (Nature Precedings : doi:10.1038/npre.2010.5373.1 : Posted 8 Dec 2010). In one embodiment, sequencing is massively parallel sequencing is of clonally amplified cfDNA molecules or of single cfDNA molecules. In another embodiment, sequencing is sequencing is massively parallel sequencing-by-synthesis with reversible dye terminators. In another embodiment, sequencing is massively parallel sequencing is performed using massively parallel sequencing-by-ligation. In another embodiment, sequencing is massively parallel sequencing is performed using massively parallel pyrosequencing. In another embodiment, sequencing is massively parallel real-time single molecule sequencing. In another embodiment, sequencing is massively parallel direct nucleotide interrogation sequencing.

The method can also be combined with assays for determining other prenatal conditions associated with the mother and/or the fetus. Examples of fetal chromosomal abnormalities include without limitation complete chromosomal trisomies or monosomies, or partial trisomies or monosomies. Examples of complete fetal trisomies include trisomy 21 (T21; Down Syndrome), trisomy 18 (T18; Edward's Syndrome), trisomy 16 (T16), trisomy 22 (T22; Cat Eye Syndrome), trisomy 15 (T15; Prader Willi Syndrome), trisomy 13 (T13; Patau Syndrome), trisomy 8 (T8; Warkany Syndrome) and the XXY (Kleinefelter Syndrome), XYY, or XXX trisomies. Examples of partial trisomies include 1q32-44, trisomy 9 p with trisomy, trisomy 4 mosaicism, trisomy 17p, partial trisomy 4q26-qter, trisomy 9, partial 2p trisomy, partial trisomy 1q, and/or partial trisomy 6p/monosomy 6q. Examples of fetal monosomies include chromosomal monosomy X, and partial monosomies such as, monosomy 13, monosomy 15, monosomy 16, monosomy 21, and monosomy 22, which are known to be involved in pregnancy miscarriage. The present method is also applicable to sequencing bioassays to determine any chromosomal abnormality if one of the parents is a known carrier of such abnormality. These include, but not limited to, mosaic for a small supernumerary marker chromosome (SMC); t(11;14)(p15;p13) translocation; unbalanced translocation t(8;11)(p23.2;p15.5); 11q23 microdeletion; Smith-Magenis syndrome 17p11.2 deletion; 22q13.3 deletion; Xp22.3 microdeletion; 10p14 deletion; 20p microdeletion, DiGeorge syndrome [del(22)(q11.2q11.23)], Williams syndrome (7q11.23 and 7q36 deletions); 1p36 deletion; 2p microdeletion; neurofibromatosis type 1 (17q11.2 microdeletion), Yq deletion ; Wolf-Hirschhorn syndrome (WHS, 4p16.3 microdeletion); 1p36.2 microdeletion; 11q14 deletion; 19q13.2 microdeletion; Rubinstein-Taybi (16 p13.3 microdeletion); 7p21 microdeletion; Miller-Dieker syndrome (17p13.3), 17p11.2 deletion; and 2q37 microdeletion.

The method can be applied to sequencing bioassays that determine the fetal fraction in a maternal sample. Determination of fetal fraction can be performed by targeting a plurality of chromosomal sequences known to comprise at least one polymorphism such as a SNP or an STR. When using SNP, the sequencing bioassay relies on using sequence-specific primers to amplify the polymorphic target sequences from fetal and maternal cfDNA in a plasma or purified nucleic acid sample, combining the amplified polymorphic target sequences with the nucleic acids of the maternal plasma sample, massively parallel sequencing the sample genomic and amplified polymorphic sequences, counting the sequence tags that align with the possible SNP sequences for each of the polymorphic sites, and determining the fetal fraction from the ratio of the number of each of the two possible mapped tags. When using STRs, the ratio of fetal and maternal STR sequences is determined by capillary electrophoresis. Methods for determining fetal fraction are described in U.S. Patent Applications Publication Nos. US2012/0010085, US2011/0224087, US2011/0201507, and US2011/0177517.

In addition to the partial and complete gain or loss of chromosomal sequences, the method is also applicable to assays that identify polymorphisms and mutations in genes implicated in disorders and in regions of the human genome that linkage and whole-genome association studies have implicated in disease. In one embodiment, the method can be applied to sequencing bioassays for determining the presence or absence of polymorphisms associated with single gene disorders. Examples of single gene disorders include without limitation autosomal dominant disorders *e.g*. familial hypercholesterolemia, hereditary spherocytosis, Marfan syndrome, neurofibromatosis type 1, hereditary nonpolyposis colorectal cancer, and hereditary multiple exostoses, and Huntington disease, autosomal recessive disorders *e.g*. Sickle cell anemia, Cystic fibrosis, Tay-Sachs disease, Tay-Sachs disease, Mucopolysaccharidoses, Glycogen storage diseases, and Galactosemia, X-linked dominant disorders *e.g.* X-linked hypophosphatemic rickets, X-linked recessive disorders *e.g.* Duchenne muscular dystrophy, hemophilia and Lesch-Nyhan syndrome, and Y-linked disorders *e.g*. male infertility and hypertrichosis pinnae.

In another embodiment, the present method can be applied sequencing bioassays for identifying polymorphisms associated with genetic disorders that are complex, multifactorial, or polygenic, meaning that they are likely associated with the effects of multiple genes in combination with lifestyle and environmental factors. Examples of polygenic disorders include without limitation polygenic disorders including but not limited to asthma, autoimmune diseases such as multiple sclerosis, cancers, ciliopathies, cleft palate, diabetes, heart disease, hypertension, inflammatory bowel disease, mental retardation, mood disorder, obesity, refractive error, and infertility.

In another embodiment, the present method can be applied to sequencing bioassays for diagnosing or determining a prognosis in a disease condition known to be associated with a specific haplotype(s), to determine novel haplotypes, and to detect haplotype associations with responsiveness to pharmaceuticals. Whole genome sequencing enables the identification of haplotypes by directly identifying the polymorphisms on a genome. In NIPD, the sequencing bioassay comprises whole genome sequencing maternal cellular DNA. Maternal cellular DNA can be obtained from a biological sample devoid of fetal genomic DNA. For example, maternal DNA can be obtained from the buffy coat layer of a maternal blood. Haplotypes comprising a plurality of polymorphic sequences that span entire chromosomes can be determined by whole genome sequencing using single molecules sequencing. The fetal haplotypes are compared to known disorder-associated haplotypes, and based on a match of the fetal haplotype with any one of the known disorder-associated haplotypes indicates that the fetus has the disorder or that the fetus is susceptible for the disorder. Fetal haplotypes can also be compared to haplotypes associated with treatment responsiveness or unresponsiveness of the specific polymorphism. Comparison of the identified fetal haplotypes to known haplotype databases allow for the diagnosis and/or prognosis of a disorder.

In another embodiment, the present method can be applied to sequencing bioassays for detecting polymorphisms associated with trinucleotide expansions *e.g*. fragile X syndrome, and polyQ diosorders such as SBMA (Spinobulbar muscular atrophy or Kennedy disease), and Spinocerebellar ataxias.

cfDNA has been found in the circulation of patients diagnosed with malignancies including but not limited to lung cancer (Pathak et al. Clin Chem 52:1833-1842 [2006]), prostate cancer (Schwartzenbach et al. Clin Cancer Res 15:1032-8 [2009]), and breast cancer (Schwartzenbach et al. available online at breast-cancer-research.com/content/11/5/R71 [2009]). Identification of genomic instabilities associated with cancers that can be determined in the circulating cfDNA in cancer patients is a potential diagnostic and prognostic tool. In some embodiments, the method is applied to bioassays that determine gene amplifications in a cancer patient. For example, the amplification of the proto-oncogene human epidermal growth factor receptor 2 (HER2) located on chromosome 17 (17(17q21-q22)), which results in overexpression of HER2 receptors on the cell surface leading to excessive and dysregulated signaling in breast cancer and other malignancies (Park et al., Clinical Breast Cancer 8:392-401 [2008]). Other examples of gene amplifications in human malignancies include c-myc in promyelocytic leukemia cell line HL60, and in small-cell lung carcinoma cell lines, N-myc in primary neuroblastomas (stages III and IV), neuroblastoma cell lines, retinoblastoma cell line and primary tumors, and small-cell lung carcinoma lines and tumors, L-myc in small-cell lung carcinoma cell lines and tumors, c-myb in acute myeloid leukemia and in colon carcinoma cell lines, c-erbb in epidermoid carcinoma cell, and primary gliomas, c-K-ras-2 in primary carcinomas of lung, colon, bladder, and rectum, N-ras in mammary carcinoma cell line (Varmus H., Ann Rev Genetics 18: 553-612 (1984) [cited in Watson et al., Molecular Biology of the Gene (4th ed.; Benjamin/Cummings Publishing Co. 1987)].

The method can also be applied to bioassays for determining chromosomal deletions involving tumor suppressor genes *e.g*. chromosomal deletion or mutation of the Rb-I gene, complete or interstitial deletions of chromosome 5, which are associated with myelodysplastic syndromes, and other chromosomal abnormalities that have been associated with various cancers.

The present invention is described in further detail in the following Examples which are not in any way intended to limit the scope of the invention as claimed. The attached Figures are meant to be considered as integral parts of the specification and description of the invention. The following examples are offered to illustrate, but not to limit the claimed invention.

### EXAMPLES

### EXAMPLE 1

### Verification of sample integrity in singleplex sequencing bioassays of clonally amplified cfDNA molecules for the determination of fetal chromosomal abnormalities

Peripheral blood samples are collected from pregnant women in their first or second trimester of pregnancy and who were deemed at risk for fetal aneuploidy. Informed consent is obtained from each participant prior to the blood draw. Blood is collected before amniocentesis or chorionic villus sampling. Karyotype analysis is performed using the chorionic villus or amniocentesis samples to confirm fetal karyotype. Approximately 6-9 ml of whole blood are drawn from each subject and collected in a blood tube comprising anticoagulant *e.g.* ACD tubes. The blood sample is centrifuged at 1600 X g at 4°C for 10.

For cell-free plasma extraction, the upper plasma layer is transferred to a 15-ml high speed centrifuge tube and centrifuged at 16000 x g, 4°C for 10 min to provide a substantially cell-free plasma containing fetal and maternal cfDNA. An antigenomic marker DNA of *e.g.* 200 bp in length is added to the cell-free plasma, and the marked cell-free plasma is stored at -80°C and thawed only once before processing in preparation of sequencing library. Samples from different individuals are each marked with a unique antigenomic sequence.

Purified cell-free DNA (cfDNA) is extracted from cell-free plasma using the QIAamp Blood DNA Mini kit (Qiagen Inc., Valencia, CA) according to the manufacturer's instruction. One milliliter of buffer AL and 100 µl of Protease solution is added to 1 ml of plasma. The mixture is incubated for 15 minutes at 56°C. One milliliter of 100% ethanol is added to the plasma digest. The resulting mixture is transferred to QIAamp mini columns that are assembled with VacValves and VacConnectors provided in the QIAvac 24 Plus column assembly (Qiagen). Vacuum is applied to the samples, and the cfDNA retained on the column filters is washed under vacuum with 750µl of buffer AW1, followed by a second wash with 750µl of buffer AW24. The column is centrifuged at 14,000 RPM for 5 minutes to remove any residual buffer from the filter. The cfDNA is eluted with buffer AE by centrifugation at 14,000 RPM, and the concentration determined using Qubit™ Quantitation Platform (Invitrogen by Life Technologies, Carlsbad, CA).

### Preparation of Sequencing Library for Singleplex Sequencing of Clonally Amplified DNA Marker and Genomic cfDNA Molecules

Marker and cfDNA molecules of the marked sample are modified in preparation of a sequencing library for sequencing using the Illumina GAII analyzer essentially according to the manufacturer's instructions. Library preparation using an aliquot of the marked sample containing approximately 2 ng of cfDNA is performed using reagents of the NEBNext™ DNA Sample Prep DNA Reagent Set 1 (Part No. E6000L; New England Biolabs, Ipswich, MA), for Illumina® as follows. Because cell-free plasma DNA is fragmented in nature, no further fragmentation by nebulization or sonication is done on the plasma DNA samples. The overhangs of the purified cfDNA and marker molecules contained in 40µl are converted into phosphorylated blunt ends according to the NEBNext® End Repair Module by incubating the cfDNA with 10X phosphorylation buffer, deoxynucleotide solution mix (10 mM each dNTP), DNA Polymerase I, T4 DNA Polymerase and T4 Polynucleotide Kinase provided in the NEBNext™ DNA Sample Prep DNA Reagent Set 1 for 15 minutes at 20°C. The enzymes are then heat inactivated by incubating the reaction mixture at 75°C for 5 minutes. The mixture is cooled to 4°C, and dA tailing of the blunt-ended DNA is accomplished using the dA-tailing master mix containing the Klenow fragment (3' to 5' exo minus) (NEBNext™ DNA Sample Prep DNA Reagent Set 1), and incubating for 15 minutes at 37°C. Subsequently, the Klenow fragment is heat inactivated by incubating the reaction mixture at 75°C for 5 minutes. Following the inactivation of the Klenow fragment, of Illumina Genomic Adaptor Oligo Mix (Part No. 1000521; Illumina Inc., Hayward, CA) is used to ligate the Illumina adaptors (Non-Index Y-Adaptors) to the dA-tailed DNA using the T4 DNA ligase provided in the NEBNext™ DNA Sample Prep DNA Reagent Set 1, by incubating the reaction mixture for 15 minutes at 25°C. The mixture is cooled to 4°C, and the adaptor-ligated cfDNA is purified from unligated adaptors, adaptor dimers, and other reagents using magnetic beads provided in the Agencourt AMPure XP PCR purification system (Part No. A63881; Beckman Coulter Genomics, Danvers, MA). Eighteen cycles of PCR are performed to selectively enrich adaptor-ligated cfDNA and marker molecules using Phusion ® High-Fidelity Master Mix (Finnzymes, Woburn, MA) and Illumina's PCR primers complementary to the adaptors (Part No. 1000537 and 1000537). The adaptor-ligated DNA is subjected to PCR (98°C for 30 seconds; 18 cycles of 98°C for 10 seconds, 65°C for 30 seconds, and 72°C for 30; final extension at 72°C for 5 minutes, and hold at 4°C) using Illumina Genomic PCR Primers (Part Nos. 100537 and 1000538) and the Phusion HF PCR Master Mix provided in the NEBNext™ DNA Sample Prep DNA Reagent Set 1, according to the manufacturer's instructions. The amplified product is purified using the Agencourt AMPure XP PCR purification system (Agencourt Bioscience Corporation, Beverly, MA) according to the manufacturer's instructions available at www.beckmangenomics.com/products/AMPureXPProtocol_000387v001.pdf. The purified amplified product is eluted in Qiagen EB Buffer, and the concentration and size distribution of the amplified libraries is analyzed using the Agilent DNA 1000 Kit for the 2100 Bioanalyzer (Agilent technologies Inc., Santa Clara, CA). The amplified DNA is sequenced using Illumina's Genome Analyzer II to obtain single-end reads of 36bp. Sequencing of library DNA is performed using the Genome Analyzer II (Illumina Inc., San Diego, CA, USA) according to standard manufacturer protocols. Copies of the protocol for whole genome sequencing using Illumina/Solexa technology may be found at BioTechniques.RTM. Protocol Guide 2007 Published December 2006: p 29, and on the world wide web at biotechniques.com/default.asp? page=protocol&subsection=article_display&id=112378. Only about 30 bp of random sequence information are needed to identify a sequence as belonging to a specific human chromosome. Longer sequences can uniquely identify more particular targets. In the present case, a large number of 36 bp reads are obtained, covering approximately 10% of the genome. The DNA library was diluted to 1nM and denatured. Library DNA (5pM) was subjected to cluster amplification according to the procedure described in Illumina's Cluster Station User Guide and Cluster Station Operations Guide, available on the world wide web at illumina.com/systems/genome analyzer/cluster_station.ilmn. Upon completion of sequencing of the sample, the Illumina "Sequencer Control Software" transferred image and base call files to a Unix server running the Illumina "Genome Analyzer Pipeline" software version 1.51. The Illumina "Gerald" program is run to align sequences of the cfDNA to the reference human genome that is derived from the hg18 genome provided by National Center for Biotechnology Information (NCBI36/hg18, available on the world wide web at http://genome.ucsc.edu/cgi-bin/hgGateway?org=Human&db=hg18&hgsid=166260105). Sequences pertaining to the marker nucleic acid are aligned to the corresponding synthetic marker sequence. The sequence data generated from the above procedure that uniquely aligned to the genome is read from Gerald output (export.txt files) by a program (c2c.pl) running on a computer running the Linnux operating system. Sequence alignments with base mis-matches are allowed and included in alignment counts only if they align uniquely to the genome. Sequence alignments with identical start and end coordinates (duplicates) are excluded.

Between about 5 and 15 million 36 bp tags with 2 or less mismatches are mapped uniquely to the human genome, and to the known sequence of the marker molecules. The sequencing information pertaining to the marker molecule is compared to the known sequence added to the source sample. The absence of a correspondence between the sequencing information and known sequence of the marker molecule is indicative of a sample mix-up, and the accompanying sequencing information pertaining to the genomic cfDNA molecules is disregarded, and no determination of chromosomal abnormality is made. The presence of a correspondence between the sequencing information and known sequence of the marker molecule verifies that the integrity of the source sample was maintained throughout the bioassay, and the presence or absence of a chromosomal abnormality *e.g*. trisomy 21, is made. Examples of methods of analyses to determine the presence or absence of chromosomal abnormalities are described for example, in U.S. Patent Application Publication 2011/0245085, Sehnert et al., Clin Chem 57:7 [2011] Published April 25, 2011 as doi:10.1373/clinchem.2011.165910, Bianchi et al., Obstetrics and Gynecol 119:5 [2012] DOI: 10.1097/AOG.obo13e31824fb482, and Fan et al. (Proc Natl Acad Sci USA 105:16266-16271 [2008], and Chiu et al., BMJ 342:c7401 [2011]).

### EXAMPLE 2

### Verification of sample integrity in singleplex sequencing bioassays of cfDNA molecules for the determination of fetal chromosomal abnormalities

A peripheral blood sample is collected, and the plasma fraction is obtained as described in Example 1. Marker molecules having identical sequences are added to the plasma fraction, which is subsequently processed to provide a purified mixture of genomic and marker molecules as described in Example 1. Marker and cfDNA molecules of the marked sample are modified in preparation of a sequencing library for sequencing using Helicos Genetic Analysis System. Marker and genomic cfDNA are treated with a terminal transferase to generate a poly-A tail, and are loaded onto the sequencer. No ligation or PCR amplification steps are required. The tailed nucleic acids hybridize to complementary poly-T strands anchored to the flow cell surface. Inside the HeliScope ™ Single Molecule Sequencer, a series of nucleotide addition and detection cycles determine the sequence of each fragment. Open source data analysis software aligns the hundreds of millions of sequence reads to the human reference genome and the known index and marker molecule sequence.

As is described above for sequencing assays of clonally amplified molecules, the sequencing information pertaining to the marker molecule is compared to the known sequence added to the source sample. The absence of a correspondence between the sequencing information and known sequence of the marker molecule is indicative of a sample mix-up, and the accompanying sequencing information pertaining to the genomic cfDNA molecules is disregarded, and no determination of chromosomal abnormality is made. The presence of a correspondence between the sequencing information and known sequence of the marker molecule verifies that the integrity of the source sample was maintained throughout the bioassay, and the presence or absence of a chromosomal abnormality *e.g*. trisomy 21, is made.

### EXAMPLE 3

### Verification of sample integrity in multiplex sequencing bioassays of clonally amplified cfDNA molecules for the determination of fetal chromosomal abnormalities

Eight maternal peripheral blood samples are each drawn into individual blood collection tubes each comprising an anticoagulant and a marker nucleic acid molecule. The marker nucleic acid used for marking each blood sample has a nucleotide sequence that is unique to each sample. The marker molecules are analogs of DNA *e.g.* phosphorothioated DNA (pDNA). The blood samples are centrifuged to separate red and white cells, and samples of purified fetal and maternal nucleic acids accompanied by the corresponding marker molecules are obtained as described in Example 1.

Marker and cfDNA molecules of the marked sample are modified in preparation of a sequencing library for sequencing using the Illumina GAII analyzer essentially according to the manufacturer's instructions. Library preparation using an aliquot of the marked sample containing approximately 2 ng of cfDNA is performed using reagents of the NEBNext™ DNA Sample Prep DNA Reagent Set 1 (Part No. E6000L; New England Biolabs, Ipswich, MA), for Illumina® as follows. Because cell-free plasma DNA is fragmented in nature, no further fragmentation by nebulization or sonication is done on the plasma DNA samples. The ends of the purified cfDNA and accompanying marker molecules blunt-ended, and dA-tailed as described in Example 1 (a). Modification of the pDNA is possible due to the compatibility of the analog with the enzymic modification processes. Following the inactivation of the Klenow fragment, Illumina Genomic Adaptor Oligo Mix (Part No. 1000521; Illumina Inc., Hayward, CA) is used to ligate the Illumina adaptors (Non-Index Y-Adaptors) to the dA-tailed DNA using the T4 DNA ligase provided in the NEBNext™ DNA Sample Prep DNA Reagent Set 1, by incubating the reaction mixture for 15 minutes at 25°C. The mixture is cooled to 4°C, and the adaptor-ligated cfDNA is purified from unligated adaptors, adaptor dimers, and other reagents using magnetic beads provided in the Agencourt AMPure XP PCR purification system (Part No. A63881; Beckman Coulter Genomics, Danvers, MA). Eighteen cycles of PCR are performed to selectively enrich adaptor-ligated cfDNA and marker molecules using Phusion ® High-Fidelity Master Mix (Finnzymes, Woburn, MA) and PCR primers comprising an indexing sequence and a sequence complementary to the PCR primer site sequence of the adaptors. The resulting library of modified and amplified cfDNA and marker molecules comprises an adaptor sequence and an index sequence, which is specific to the library of each sample.

The eight sequencing libraries are pooled, and subjected to cluster amplification. The mixture of clonally amplified nucleic acids the 8 libraries is sequenced as described in Example 1. Mapping of the resulting sequence reads is performed using a human reference genome and a synthetic genome comprising index and marker molecule sequences. Sequence tags associated with identical index sequences are grouped to associate the genomic and marker sequences and distinguish sequences belonging to each library. Analysis of the grouped marker and genomic sequences is then performed to verify that the sequence obtained for the marker molecule corresponds to the known sequence added to the source sample. The absence of a correspondence between the sequencing information and known sequence of the marker molecule is indicative of a sample mix-up, and the accompanying sequencing information pertaining to the genomic cfDNA molecules is disregarded, and no determination of chromosomal abnormality is made. The presence of a correspondence between the sequencing information and known sequence of the marker molecule verifies that the integrity of the source sample was maintained throughout the bioassay, and the presence or absence of a chromosomal abnormality *e.g*. trisomy 21, is made.

### EXAMPLE 4

### Verification of sample integrity in multiplex single molecule sequencing cfDNA molecules for the determination of fetal chromosomal abnormalities

Eight maternal peripheral blood samples are drawn into separate blood collection tubes, each containing an anticoagulant and a marker nucleic acid molecule. The marker nucleic acid used for marking each blood sample has a nucleotide sequence that is unique to each sample. The marker molecules are PNA analogs of DNA. The marker and genomic nucleic acids are purified as described in the previous examples. PNA analogs cannot be modified by enzymes used to end-repair and dA-tail nucleic acids, but are amplifiable in PCR. Thus, the PNA marker molecules used for multiplex parallel sequencing are synthesized to comprise an index sequence and a sequence that is complementary to the sequence of the oligonucleotide anchored on the flow cell of the sequencer. In this example, PNA molecules comprise distinct index sequences and a polyA tail that is complementary to the polyT flow cell anchor.

A polyA tail is added to the genomic DNA in each sample, and mixtures of genomic and marker molecules from each sample are combined and are loaded onto the sequencer. No ligation or PCR amplification steps are required. The tailed nucleic acids hybridize to complementary poly-T strands anchored to the flow cell surface. Inside the HeliScope ™ Single Molecule Sequencer, a series of nucleotide addition and detection cycles determine the sequence of each fragment. Open source data analysis software aligns the hundreds of millions of sequence reads to the human reference genome and the known index and marker molecule sequence.

Analysis of sequence tags is performed as described in Example 3, and the presence or absence of a chromosomal abnormality is determined only if a correspondence is established between the sequence information of the marker molecules and the known sequence of the marker that was added to the blood collection tube and used to mark the sample.

### EXAMPLE 5

### Verification of sample integrity in multiplex bioassays of maternal cfDNA

Marker molecules of sequences known not be contained in any known genome were synthesized and used to verify the integrity of whole blood and of plasma maternal source samples that were processed to extract and sequence the mixture of fetal and maternal cfDNA in the maternal samples.

Current and previous experimental data have shown that the average length of cfDNA is about 170bp. Antigenomic sequences of 170bp were identified for their absence in any of the known genomes using BLAST searches against all genome entries. Six marker molecules (MM1-MM6) were synthesized based on the sequences of the identified antigenomic sequences (SEQ ID NO:1-6; Table 1), and were used to verify the integrity of the samples as follows.

**TABLE 1**

| **Marker Molecules** | |
|---|---|
| **Marker Molecule** | **Antigenomic Sequence** |
| **MM1** | gcacatcccgctccgggtgactattaaagacgaccctcgatcatagcactcgatcagattgtgacgtatgatctgtaggacatacttcttggcc actaaccagacggtgcgagatatttcgaattgcgcctacctatctggaacgactaatgtcaattcttcgaatgaca (SEQ ID NO:1) |
| **MM2** | cgccaatcgcgctctatgcttaacgcacgtcctgtctctttatagagataccgtgggtgacggcgtgaccgggagccttgaggagagcataa agcgtaaccggattatcccgaatggtatatgacggtccctcgcataccggaccgggcattactcagcagcggttctgc (SEQ ID NO:2) |
| **MM3** | ccccaatagtgcggtgatctaacacctgacatcgggccgaaagaggaattaagagccgaccggctagactgcccatgtgccaaatcaggg gtcgaggaggttgtgtggcgacatcctattggttccacctggcggaatcgggcaaagccaccatcactggactgagaacc (SEQ ID NO:3) |
| **MM4** | agtccagtaattgcgaggaaccacttactcggtacaccgctcctggctggggttggcagaccagtcatgttgctgaggaccgacgaccccg gaccatttaactctcagacgtaccgacagcaactttgccgaattctctccagcaatcgagagcgggaaggcataagtgc (SEQ ID NO:4) |
| **MM5** | agaaccatctccggcgcaagtctacgcgagttggccttagctcatacctacggatgtggaggataagtccttagctcgtaccatcgtaaccta gtggcgtcatgcgcctacgtgagaaggattctttactgagcgcagagttgtccgtctactgccacgggccataacgc (SEQ ID NO:5) |
| **MM6** | cctaaggcctacttcaatatcgtgatgcacccgaatgactaaaggggtatatggagtatgtccatggcgtcattgagcccgcttaggatctact gtaatccgagggatacatgcctcacgcgagtctttcctaccgctactagacattatggtgcgcgccttgagtacgt (SEQ ID NO:6) |

Peripheral blood was drawn from a pregnant female into 4 blood collection tubes, (Cell-Free DNA™ BCT, Streck, Inc. Omaha NE) and shipped overnight to the laboratory for analysis. Two whole blood source samples were spiked with marker molecules as follows. One blood source sample was spiked with 720pg of marker molecule 1 (MM1) and a second blood source sample was spiked with 720pg of Marker Molecule 2. All 4 tubes were centrifuged at 1600g for 10 minutes at 4°C. The plasma supernatant was removed from each of the four tubes and placed into 5mL high speed centrifuge tubes and centrifuged at 16000g for 10 minutes at 4°C. The plasma fractions of the whole blood that had been spiked with marker molecules were aliquoted into separate tubes and stored at -80°C. Plasma fractions from the two remaining blood tubes (unspiked) were pooled then divided into 1.1mL aliquots. Plasma source sample samples were prepared as follows. One hundred picograms of MM1 were added to one plasma aliquot, 100pg of MM2 were added to plasma aliquot 2 etc to obtain 6 marked plasma source samples each containing a different marker molecule (MM1-MM6) stored at - 80°C.

One tube of each marked plasma source sample and 1 tube of each marked source blood sample were thawed and DNA was extracted using the Qiagen Blood Mini Kit according to the method described in example 1. Thirty microliters of each sample DNA was used to prepare a library using the TruSeq™ DNA Sample Preparation Kit containing indexes 1-6 (Illumina®, San Diego, CA). Sequencing libraries were prepared such that samples containing MM1 were indexed using index molecule 1, samples containing MM2 were indexed using index 2 etc. The sequencing libraries were quantified using the Agilent Bioanalyzer DNA1000 Kit (Agilent Technologies, Santa Clara, CA) and diluted to 4nM with Qiagen buffer EB. The indexed and marked samples were pooled and further diluted to 2nM before being sequenced in four lanes of an Illumina HiSeq flow-cell using the Illumina TruSeq SBS kit v3 according to Table 2.

**TABLE 2**

| **Multiplexed Sequencing Flow-cell Layout** | | | | | | |
|---|---|---|---|---|---|---|
| Lane | Index 1 | Index 2 | Index 3 | Index 4 | Index 5 | Index 6 |
| 1 | Plasma source/MM1 | Plasma source/MM2 | Plasma source/MM3 | Plasma source/MM4 | Plasma source/MM5 | Plasma source/MM6 |
| 2 | Blood source/MM1 | Blood source/MM2 | Plasma source/MM3 | Plasma source/MM4 | Plasma source/MM5 | Plasma source/MM6 |
| 3 | Plasma source/MM1 | Plasma source/MM2 | Plasma source/MM3 | Plasma source/MM4 | Plasma source/MM5 | Plasma source/MM6 |
| 4 | Blood source/MM1 | Blood source/MM2 | Plasma source/MM3 | Plasma source/MM4 | Plasma source/MM5 | Plasma source/MM6 |

Sequence reads were aligned to the human reference genome hg19 and to a synthetic reference genome comprising the sequences of the antigenomic marker molecules. Sequence reads that mapped uniquely *i.e.* only once, to the hg19 reference genome or to the synthetic reference genome of marker molecule sequences were counted (Table 3).

**TABLE 3**

| **Correspondence of MM Sequence with Source Sample cfDNA Sequence** | | | | | | | | | | |
|---|---|---|---|---|---|---|---|---|---|---|
| **Sample** | **I*** | **L**** | **Indexed Reads** | **MM1** | **MM2** | **MM3** | **MM4** | **MM5** | **MM6** | **% MM reads** |
| Plasma /MM1 | 1 | 1 | 29538429 | 141477 | 21 | 92 | 137 | 34 | 104 | 0.48 |
| Plasma /MM2 | 2 | 1 | 38975166 | 21 | 134900 | 144 | 196 | 23 | 108 | 0.35 |
| Plasma /MM3 | 3 | 1 | 29584211 | 22 | 20 | 439692 | 209 | 21 | 78 | 1.49 |
| Plasma /MM4 | 4 | 1 | 37552314 | 43 | 31 | 116 | 463015 | 55 | 148 | 1.23 |
| Plasma /MM5 | 5 | 1 | 31028884 | 28 | 34 | 131 | 151 | 114867 | 88 | 0.37 |
| Plasma /MM6 | 6 | 1 | 33462858 | 45 | 44 | 135 | 176 | 32 | 267112 | 0.80 |
| Blood /MM1 | 1 | 2 | 25467176 | 200800 | 16 | 77 | 89 | 20 | 59 | 0.79 |
| Blood /MM2 | 2 | 2 | 31517302 | 54 | 90429 | 113 | 130 | 29 | 60 | 0.29 |
| Plasma /MM3 | 3 | 2 | 25221978 | 45 | 9 | 325135 | 145 | 17 | 56 | 1.29 |
| Plasma source/ MM4 | 4 | 2 | 31058300 | 48 | 21 | 84 | 328159 | 24 | 82 | 1.06 |
| Plasma /MM5 | 5 | 2 | 26307540 | 40 | 19 | 78 | 105 | 83421 | 52 | 0.32 |
| Plasma /MM6 | 6 | 2 | 27427098 | 33 | 8 | 95 | 106 | 22 | 189294 | 0.69 |
| Plasma /MM1 | 1 | 3 | 30167594 | 124292 | 24 | 84 | 130 | 25 | 70 | 0.41 |
| Plasma /MM2 | 2 | 3 | 37167426 | 41 | 115311 | 132 | 159 | 29 | 85 | 0.31 |
| Plasma /MM3 | 3 | 3 | 27936334 | 23 | 18 | 369974 | 175 | 23 | 71 | 1.32 |
| Plasma /MM4 | 4 | 3 | 36550452 | 36 | 27 | 117 | 398467 | 34 | 104 | 1.09 |
| Plasma /MM5 | 5 | 3 | 29121964 | 32 | 29 | 94 | 138 | 94604 | 67 | 0.32 |
| Plasma /MM6 | 6 | 3 | 31480407 | 46 | 25 | 128 | 160 | 28 | 218423 | 0.69 |
| Blood /MM1 | 1 | 4 | 30305516 | 306564 | 25 | 125 | 147 | 30 | 101 | 1.01 |
| Blood /MM2 | 2 | 4 | 40195116 | 83 | 142218 | 184 | 222 | 22 | 119 | 0.35 |
| Plasma /MM3 | 3 | 4 | 31091454 | 60 | 25 | 494827 | 230 | 26 | 100 | 1.59 |
| Plasma /MM4 | 4 | 4 | 40152887 | 65 | 33 | 149 | 525766 | 45 | 127 | 1.31 |
| Plasma /MM5 | 5 | 4 | 31170622 | 66 | 19 | 122 | 167 | 120584 | 88 | 0.39 |
| Plasma /MM6 | 6 | 4 | 33483476 | 110 | 33 | 139 | 159 | 33 | 281742 | 0.84 |

| | | | | | | | | | | |
|---|---|---|---|---|---|---|---|---|---|---|
| *I= Index **L=Lane | | | | | | | | | | |

The data show that for every sample, the sequence of the MM that had been added to the source sample was determined only in correspondence with the sequence of the cfDNA of the source sample to which the MM had been added. For example, the data for Sample 1 show that the sequence of the reads mapping to MM1 were determined only in correspondence with the sequence of the cfDNA that had been obtained from the source sample (plasma sample 1) to which MM1 had been added. In addition, the absence of a different sequence *e.g*. MM2, from the reads obtained from sequencing cfDNA of source sample 1, shows the absence of cross contamination of source sample 1 by another sample *e.g*. source sample 2.

### EXAMPLE 6

### Verification of Non-Biological Samples identity using real-time PCR

The method disclosed herein can be used to verify the identity of source samples that are not of biological origin, *i.e.* marker molecules that are verifiable by sequencing can be incorporated into source samples whose identity is not verifiable by DNA analysis as exemplified as follows.

During the process of manufacture of pharmaceutical products *e*.*g*. pills, tablets or capsules, lyophilized marker molecules are added to the mixture of pharmacological ingredients of the pills, and are incorporated into the final product. Different marker molecules are used for different products and/or different batches of the products. The marker molecules are extracted and analyzed to provide evidence of an unbroken chain of identification and verification of the marker molecule throughout the products' entire manufacturing and distribution path. Similarly, marker molecules are used to track and verify the authenticity of the product in cases of suspected product tampering or illegal reproduction.

Verification of the integrity of the product is performed by determining the sequence of the marker molecule used in conjunction with product manufacturing.

The sequence of the marker molecule is determined using real-time PCR utilizing probes having sequences corresponding to those of markers used in the manufacture of various batches of the product. Fluorescent signal is detected for the probe corresponding to the marker used in the batch of pharmaceuticals to verify the origin of the product.

## Claims

1. A method for verifying the integrity of a plurality of biological source samples comprising genomic nucleic acids and determining the presence or absence of at least one fetal chromosomal abnormality in each of said plurality of biological source samples, said method comprising:
(a) combining a unique marker nucleic acid with each of said plurality of biological source samples, wherein said plurality of biological source samples are maternal blood or plasma samples that comprise fetal and maternal cell-free DNA, thereby obtaining a plurality of uniquely marked samples each comprising a unique mixture of genomic and marker nucleic acids;
(b) incorporating distinct indexing sequences into said genomic and marker nucleic acids of each of said uniquely marked samples thereby providing a uniquely marked indexed mixture of indexed marker and indexed sample nucleic acids for each of said plurality of source samples;
(c) massively parallel sequencing a combination of uniquely marked indexed mixtures of indexed nucleic acids by multiplex sequencing; and
(d) determining a correspondence between the sequence of said indexed marker and the sequence of said indexed genomic nucleic acids obtained in step (c) for each of said uniquely marked indexed mixtures of nucleic acids in said combination and the sequence of said unique marker nucleic acid in each of said uniquely marked samples, thereby verifying the integrity of each of said plurality of biological source samples,
and determining the presence or absence of at least one fetal chromosomal abnormality in each of said plurality of marked indexed samples.

2. The method of Claim 1, further comprising isolating said unique mixture of genomic and marker nucleic acids for each of said plurality of samples.

3. The method of any preceding claim, wherein said at least one fetal chromosomal abnormality is:
(i) selected from a partial chromosomal aneuploidy, a complete chromosomal aneuploidy, and a polymorphism; or
(ii) associated with a disorder.

4. The method of any preceding claim, wherein said plurality of source samples are obtained from different pregnant female subjects.

5. The method of any preceding claim, wherein (i) said marker nucleic acid is DNA or an analog thereof; and/or (ii) the unique marker molecules in a sample are a combination of two, three, four, five, six, seven, eight, nine, ten, fifteen, twenty, or more different sequences.

6. The method of any preceding claim, wherein the identity of a sample is verified using a plurality of marker nucleic acid molecules that have at least two, at least three, at least four, at least five, at least six, at least seven, at least eight, at least nine, at least ten, at least 15, at least 20, at least 30, at least 40, or at least 50, different sequences.

7. The method of any of claims 1-6, wherein the marker nucleic acid molecules are added to unprocessed or partially processed source samples.

8. The method of any of claims 1-6, wherein the marker nucleic acid molecules are added to a purified sample.

9. The method of any of claims 1-8, wherein the marker nucleic acid molecules are added to a fraction of the sample.

10. The method of any preceding claim, wherein:
(i) the marker nucleic acid molecules are at least 30bp in length;
(ii) the length of the marker nucleic acids is up to 600bp;
(iii) the length of the marker nucleic acids is about 150bp, about 160bp, 170bp, about 180bp, about 190bp, or about 200bp;
(iv) the length of the marker nucleic acids is about 170bp; or
(v) the length of the marker nucleic acids is between about 100bp and 600bp.

11. The method of any preceding claim, wherein the distinct indexing sequences are distinct base sequences of about 5, about 10, about 15, about 20, about 25, or more, bases that are added at the 3' end of the genomic and marker nucleic acids.

12. The method of any preceding claim, wherein said massively parallel sequencing is of clonally amplified cfDNA molecules or of single cfDNA molecules.

13. The method of any preceding claim, wherein said massively parallel sequencing is:
(a) massively parallel sequencing-by-synthesis;
(b) performed using massively parallel sequencing-by-ligation
(c) massively parallel pyrosequencing;
(d) massively parallel direct nucleotide interrogation sequencing; or
(e) ion semiconductor sequencing.

## Patentansprüche

1. Verfahren zum Verifizieren der Integrität einer Mehrzahl von Proben von biologischem Ausgangsmaterial umfassend genomische Nukleinsäuren, und Bestimmen des Vorhandenseins oder Fehlens von mindestens einer fötalen chromosomalen Abnormität in jeder der Mehrzahl von Proben von biologischem Ausgangsmaterial, wobei das Verfahren Folgendes umfasst:
(a) Kombinieren einer einzigartigen Markernukleinsäure mit jeder der Mehrzahl von Proben von biologischem Ausgangsmaterial, wobei es sich bei der genannten Mehrzahl von Proben von biologischem Ausgangsmaterial um mütterliche Blut- oder Plasmaproben, die fötale und mütterliche zellfreie DNA umfassen, handelt, wodurch man eine Mehrzahl von einzigartig markierten Proben erhält, die jeweils eine einzigartige Mischung aus genomischen Nukleinsäuren und Markernukleinsäuren umfassen;
(b) Einbauen von unverwechselbaren Indexingsequenzen in die genomischen Nukleinsäuren und die Markernukleinsäuren von jeder der einzigartig markierten Proben, wodurch man zu einer einzigartig markierten indexierten Mischung von indexierten Markernukleinsäuren und indexierten Probennukleinsäuren für jede der Mehrzahl von Proben von Ausgangsmaterial bereitstellt;
(c) massives paralleles Sequenzieren einer Kombination von einzigartig markierten indexierten Mischungen von indexierten Nukleinsäuren mittels Multiplex-Sequenzieren; und
(d) Bestimmen einer Übereinstimmung zwischen der Sequenz des indexierten Markers und der Sequenz der in Schritt (c) erhaltenen indexierten genomischen Nukleinsäuren für jede der einzigartig markierten indexierten Mischungen von Nukleinsäuren in der Kombination und der Sequenz der einzigartigen Markernukleinsäure in jeder der einzigartig markierten Proben, wodurch man die Integrität von jeder der Mehrzahl von Proben von biologischem Ausgangsmaterial verifiziert,
und Bestimmen des Vorhandenseins oder Fehlens von mindestens einer fötalen chromosomalen Abnormität in jeder der Mehrzahl von markierten indexierten Proben.

2. Verfahren nach Anspruch 1, das weiterhin umfasst: Isolieren der einzigartigen Mischung von genomischen Nukleinsäuren und Markernukleinsäuren für jede der Mehrzahl von Proben.

3. Verfahren nach einem vorhergehenden Anspruch, wobei die mindestens eine fötale chromosomale Abnormität:
(i) aus einer partialen chromosomalen Aneuploidie einer vollständigen chromosomalen Aneuploidie und einem Polymorphismus ausgewählt ist; oder
(ii) mit einer Krankheit assoziiert ist.

4. Verfahren nach einem vorhergehenden Anspruch, wobei die Mehrzahl von Proben von Ausgangsmaterial von unterschiedlichen schwangeren weiblichen Individuen stammt.

5. Verfahren nach einem vorhergehenden Anspruch, wobei (i) es sich bei der Markernukleinsäure um DNA oder ein Analog davon handelt; und/oder (ii) die einzigartigen Markermoleküle in einer Probe eine Kombination von 2, 3, 4, 5, 6, 7, 8, 9, 10, 15, 20 oder mehr unterschiedlichen Sequenzen sind.

6. Verfahren nach einem vorhergehenden Anspruch, wobei die Identität einer Probe unter Verwendung einer Mehrzahl von Markernukleinsäuremolekülen verifiziert wird, die mindestens zwei, mindestens drei, mindestens vier, mindestens fünf, mindestens sechs, mindestens sieben, mindestens acht, mindestens neun, mindestens zehn, mindestens 15, mindestens 20, mindestens 30, mindestens 40 oder mindestens 50 unterschiedliche Sequenzen aufweisen.

7. Verfahren nach einem der Ansprüche 1-6, wobei die Markernukleinsäuremoleküle zu unverarbeiteten oder teilverarbeiteten Proben von Ausgangsmaterial zugesetzt werden.

8. Verfahren nach einem der Ansprüche 1-6, wobei die Markernukleinsäuremoleküle einer gereinigten Probe zugesetzt werden.

9. Verfahren nach einem der Ansprüche 1-8, wobei die Markernukleinsäuremoleküle zu einer Fraktion der Probe zugesetzt werden.

10. Verfahren nach einem vorhergehenden Anspruch, wobei:
(i) die Markernukleinsäure eine Länge von mindestens 30 Bp aufweisen;
(ii) die Länge der Markernukleinsäuren bis zu 600 Bp beträgt;
(iii)die Länge der Markernukleinsäuren ungefähr 150 Bp beträgt; ungefähr 160 Bp, 170 Bp, ungefähr 180 Bp, ungefähr 190 Bp oder ungefähr 200 Bp beträgt;
(iv) die Länge der Markernukleinsäuren ungefähr 170 Bp beträgt; oder
(v) die Länge der Markernukleinsäuren zwischen ungefähr 100 Bp und 600 Bp beträgt.

11. Verfahren nach einem vorhergehenden Anspruch, wobei es sich bei den unverwechselbaren Indexingsequenzen um unverwechselbare Basensequenzen mit ungefähr 5, ungefähr 10, ungefähr 15, ungefähr 20, ungefähr 25 oder mehr Basen, die am 3'-Ende der genomischen Nukleinsäuren und der Markernukleinsäuren zugefügt sind, handelt.

12. Verfahren nach einem vorhergehenden Anspruch, wobei das massive parallele Sequenzieren das Sequenzieren von klonal amplifizierten cfDNA-Molekülen oder von einzelnen cfDNA-Molekülen ist.

13. Verfahren nach einem vorhergehenden Anspruch, wobei das massive parallele Sequenzieren
(a) massives paralleles Sequenzieren durch Synthese (sequencing-by-synthesis) ist;
(b) unter Verwendung von massivem parallelem Sequenzieren durch Ligieren (sequencing-by-ligation) durchgeführt wird;
(c) massives paralleles Pyrosequenzieren ist;
(d) massives paralleles direktes Nukleotidabfragesequenzieren ist; oder
(e) Ionenhalbleitersequenzieren ist.

## Revendications

1. Méthode de vérification de l'intégrité d'une multitude d'échantillons de source biologique comprenant des acides nucléiques génomiques et de détermination de la présence ou de l'absence d'au moins une anomalie chromosomique foetale dans chacun des échantillons de ladite multitude d'échantillons de source biologique, ladite méthode comprenant :
(a) la combinaison d'un acide nucléique marqueur unique avec chacun des échantillons de ladite multitude d'échantillons de source biologique, où ladite multitude d'échantillons de source biologique est constituée d'échantillons de plasma ou de sang maternel qui comprennent de l'ADN foetal et maternel hors cellule, obtenant ainsi une multitude d'échantillons à marquage unique, chacun comprenant un mélange unique d'acides nucléiques génomiques et marqueurs ;
(b) l'incorporation de séquences d'indexation distinctes dans lesdits acides nucléiques génomiques et marqueurs de chacun desdits échantillons à marquage unique, pour obtenir un mélange indexé à marquage unique d'acides nucléiques marqueurs indexés et d'échantillons indexés pour chacun des échantillons de ladite multitude d'échantillons de source ;
(c) le séquençage parallèle à grande échelle d'une combinaison de mélanges indexés à marquage unique d'acides nucléiques indexés par séquençage multiplexé ; et
(d) la détermination d'une correspondance entre la séquence dudit marqueur indexé et la séquence desdits acides nucléiques génomiques indexés obtenus dans l'étape (c) pour chacun desdits mélanges indexés à marquage unique d'acides nucléiques dans ladite combinaison et la séquence dudit acide nucléique marqueur unique de chacun desdits échantillons à marquage unique, vérifiant ainsi l'intégrité de chacun des échantillons de ladite multitude d'échantillons de source biologique,
et la détermination de la présence ou de l'absence d'au moins une anomalie chromosomique foetale dans chacun des échantillons de ladite multitude d'échantillons indexés marqués.

2. Méthode selon la Revendication 1, comprenant en outre l'isolement dudit mélange unique d'acides nucléiques génomiques et marqueurs pour chacun des échantillons de ladite multitude d'échantillons.

3. Méthode selon l'une quelconque des revendications précédentes, où l'au moins une anomalie chromosomique foetale est :
(i) choisie parmi une aneuploïdie chromosomique partielle, une aneuploïdie chromosomique complète et un polymorphisme ; ou
(ii) associée à un trouble.

4. Méthode selon l'une quelconque des revendications précédentes, où ladite multitude d'échantillons de source est obtenue à partir de différentes femmes enceintes.

5. Méthode selon l'une quelconque des revendications précédentes, où (i) ledit acide nucléique marqueur est l'ADN ou l'un de ses analogues ; et/ou (ii) les molécules marqueurs uniques au sein d'un échantillon sont une combinaison de deux, trois, quatre, cinq, six, sept, huit, neuf, dix, quinze, vingt séquences différentes ou plus.

6. Méthode selon l'une quelconque des revendications précédentes, où l'identité d'un échantillon est vérifiée en utilisant une multitude de molécules d'acides nucléiques marqueurs comportant au moins deux, au moins trois, au moins quatre, au moins cinq, au moins six, au moins sept, au moins huit, au moins neuf, au moins dix, au moins 15, au moins 20, au moins 30, au moins 40, ou au moins 50 séquences différentes.

7. Méthode selon l'une quelconque des revendications 1 à 6, où les molécules d'acides nucléiques marqueurs sont ajoutées à des échantillons de source traitée ou partiellement traitée.

8. Méthode selon l'une quelconque des revendications 1 à 6, où les molécules d'acides nucléiques marqueurs sont ajoutées à un échantillon purifié.

9. Méthode selon l'une quelconque des revendications 1 à 8, où les molécules d'acides nucléiques marqueurs sont ajoutées à une fraction de l'échantillon.

10. Méthode selon l'une quelconque des revendications précédentes, où :
(i) les molécules d'acides nucléiques marqueurs sont longues d'au moins 30 bp ;
(ii) la longueur des acides nucléiques marqueurs peut atteindre 600 bp ;
(iii) la longueur des acides nucléiques marqueurs est d'environ 150 bp, d'environ 160 bp, de 170 bp, d'environ 180 bp, d'environ 190 bp, ou d'environ 200 bp ;
(iv) la longueur des acides nucléiques marqueurs est d'environ 170 bp ; ou
(v) la longueur des acides nucléiques marqueurs est comprise entre environ 100 bp et 600 bp.

11. Méthode selon l'une quelconque des revendications précédentes, où les séquences d'indexation distinctes sont des séquences de bases distinctes d'environ 5, d'environ 10, d'environ 15, d'environ 20, d'environ 25 bases ou plus, qui sont ajoutées à l'extrémité 3' des acides nucléiques génomiques et marqueurs.

12. Méthode selon l'une quelconque des revendications précédentes, où ledit séquençage parallèle à grande échelle concerne des molécules de cfADN amplifié par clonage ou de molécules de cfADN uniques.

13. Méthode selon l'une quelconque des revendications précédentes, où ledit séquençage parallèle à grande échelle est :
(a) un séquençage parallèle par synthèse à grande échelle ;
(b) mis en oeuvre en utilisant un séquençage parallèle par ligature à grande échelle
(c) un pyroséquençage parallèle à grande échelle ;
(d) un séquençage par interrogation nucléotidique directe parallèle à grande échelle ; ou
(e) un séquençage par semi-conducteurs ioniques.
